(19) Europäisches Patentamt
European Patent Office
Office européen des brevets

(11) Veröffentlichungsnummer : **0 370 236 B1**

(12) # EUROPÄISCHE PATENTSCHRIFT

(45) Veröffentlichungstag der Patentschrift : **27.12.91 Patentblatt 91/52**

(51) Int. Cl.$^5$ : **C07D 487/14,** A61K 31/40, // (C07D487/14, 209:00, 209:00, 209:00)

(21) Anmeldenummer : **89119537.2**

(22) Anmeldetag : **20.10.89**

(54) **Indolocarbazol-Derivate, Verfahren zu deren Herstellung und deren Verwendung als Arzneimittel.**

(30) Priorität : **21.10.88 DE 3835842**

(43) Veröffentlichungstag der Anmeldung : **30.05.90 Patentblatt 90/22**

(45) Bekanntmachung des Hinweises auf die Patenterteilung : **27.12.91 Patentblatt 91/52**

(84) Benannte Vertragsstaaten : **AT BE CH DE ES FR GB GR IT LI LU NL SE**

(56) Entgegenhaltungen :
**EP-A- 0 269 025**
**TETRAHEDRON LETTERS, Band 24, 1983 I.-HUGHES et al. "Synthesis of Arcyriaflavin B" Seiten 1441-1444**
**THE JOURNAL OF ORGANIC CHEMISTRY, Band 52, 1987 R.P.JOYCE et al. "Synthesis of the Aromatic and Mono- saccharide Moieties of Staurosporine" Seiten 1177-1185**

(73) Patentinhaber : **GÖDECKE AKTIENGESELLSCHAFT
Salzufer 16
W-1000 Berlin 10 (DE)**

(72) Erfinder : **Kleinschroth, Jürgen, Dr.
Ricarda-Huch-Strasse 11
W-7819 Denzlingen (DE)**
Erfinder : **Barth, Hubert, Dr.
Bertold-Brecht-Weg 6
W-7830 Emmendingen (DE)**
Erfinder : **Hartenstein, Johannes, Dr.
Fohrenbühl 23
W-7801 Stegen-Wittental (DE)**
Erfinder : **Schächtele, Christoph, Dr.
Darriwald 16
W-7800 Freiburg (DE)**
Erfinder : **Rudolph, Claus, Dr.
Riedmattenstrasse 11
W-7801 Vörstetten (DE)**
Erfinder : **Osswald, Hartmut, Prof. Dr.
Kiefernweg 1
W-7808 Waldkirch 2 (DE)**

EP 0 370 236 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue Indolocarbazol-Derivate der allgemeinen Formel I

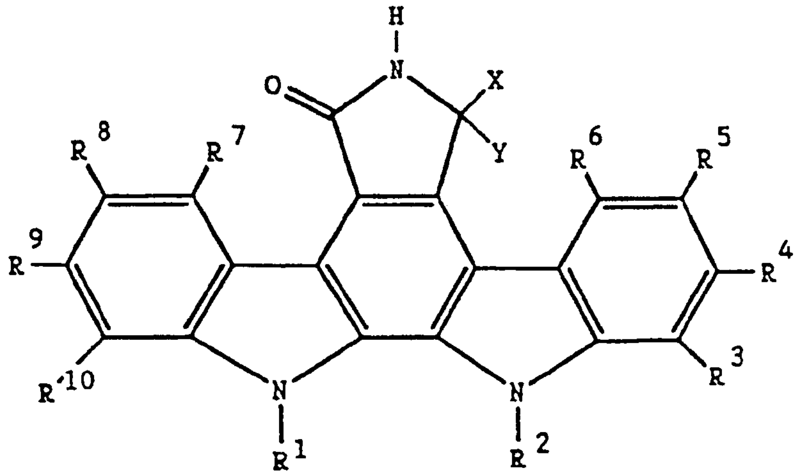

(I)

in welcher $R^1$ und $R^2$ gleich oder verschieden sind und Wasserstoff, eine geradkettige oder verzweigte Alkylgruppe mit 1 bis 18 C-Atomen, eine Cyanoalkylgruppe mit 2 bis 4 C-Atomen, eine unsubstituierte oder mit bis zu drei $C_{1-4}$-Alkylgruppen, $C_{1-4}$-Alkoxygruppen oder Halogenatomen substituierte Benzylgruppe, eine Aminoalkylgruppe mit bis zu 12 C-Atomen, am Stickstoffatom unsubstituiert oder mono- oder disubstituiert durch Benzyl oder Alkylreste mit 1 bis 4 C-Atomen oder bei der zwei Substituenten am Stickstoffatom zusammen mit dem Stickstoffatom oder ein Substituent am Stickstoffatom und eine Substituent der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, wobei die Alkylkette durch weitere $C_{1-4}$-Alkylreste, eine Hydroxygruppe oder eine $C_{1-4}$-Alkoxygruppe substituiert sein kann, einen Alkoxycarbonylalkylrest mit bis zu 7 C-Atomen, einen Rest—$CH_2$-CO-$NR^{11}R^{12}$, bei dem $R^{11}$ und $R^{12}$ gleich oder verschieden sind und für Wasserstoff, eine Alkylgruppe mit 1 bis 6 C-Atomen oder eine Benzylgruppe stehen, einen Halogenalkyl-, Hydroxyalkyl- oder Alkoxyalkylrest mit jeweils bis zu 6 C-Atomen, eine Acylgruppe mit 1 bis 4 C-Atomen oder $R^1$ und $R^2$ zusammen eine Alkylengruppe mit 2 bis 4 C-Atomen, die gegebenenfalls durch eine Hydroxygruppe, eine Alkoxygruppe mit 1 bis 4 C-Atomen oder eine unsubstituierte oder eine durch Benzyl oder Alkyl mit 1 bis 4 C-Atomen mono- oder disubstituierte Aminogruppe substituiert sein kann, bedeuten, X und Y entweder gleich sind und beide jeweils Wasserstoff bedeuten oder X und Y verschieden sind, wobei einer der Reste X oder Y für Wasserstoff steht und der andere von den beiden Resten eine Hydroxy- oder eine Alkoxygruppe mit 1 bis 4 C-Atomen bedeutet, $R^3$, $R^4$, $R^5$, $R^6$, $R^7$, $R^8$, $R^9$ und $R^{10}$ unabhängig voneinander Wasserstoff, $C_{1-4}$-Alkyl, $C_{1-4}$-Alkoxy, $C_{1-4}$-Acyl, Halogen, insbesondere Chlor oder Brom, eine Nitrogruppe, eine unsubstituierte oder durch Benzyl oder Alkylreste mit 1 bis 4 C-Atomen mono- oder disubstituierte Aminogruppe, eine Benzyloxygruppe, eine Hydroxygruppe, eine Aminoalkoxygruppe mit bis zu 12 C-Atomen, am Stickstoffatom unsubstituiert oder mono- oder disubstituiert durch Benzyl oder Alkylreste mit 1 bis 4 C-Atomen oder bei der zwei Substituenten oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, wobei die Alkylkette durch weitere $C_{1-4}$-Alkylreste, eine Hydroxygruppe oder eine $C_{1-4}$-Alkoxygruppe substituiert sein kann, eine Trifluormethylgruppe oder zwei benachbarte Reste zusammen eine Methylendioxygruppe bedeuten, mit der Maßgabe, daß mindestens einer und bis zu vier der Reste $R^3$ bis $R^{10}$ von Wasserstoff verschieden sind, deren pharmakologisch unbedenkliche Salze, sowie Arzneimittel mit einem Gehalt an mindestens einer der Verbindungen I.

Die Erfindung betrifft auch Verfahren zur Herstellung der Verbindungen der allgemeinen Formel I oder von Regioisomerengemischen dieser Verbindungen I, dadurch gekennzeichnet, daß man entweder

A) Imide der allgemeinen Formel II,

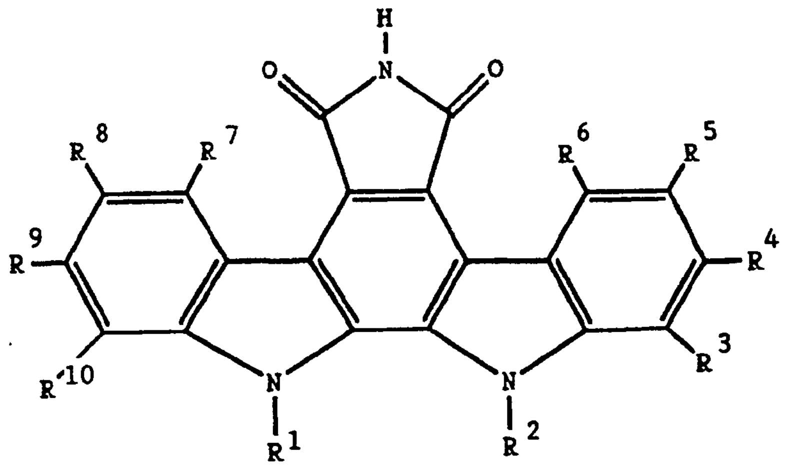

(II)

in welcher $R^1$ bis $R^{10}$ die oben genannte Bedeutung haben, mit Zink oder Zinkamalgam in Gegenwart einer organischen oder anorganischen Säure zu Verbindungen der allgemeinen Formel I, worin X und Y Wasserstoff bedeuten, reduziert, oder

B) Imide der allgemeinen Formel II, in welcher $R^1$ bis $R^{10}$ die oben genannte Bedeutung haben, mit Zink oder Zinkamalgam in Gegenwart einer organischen oder anorganischen Säure, mit Borhydriden oder mit Lithiumaluminiumhydrid zu Verbindungen der allgemeinen Formel I, in welcher X oder Y eine Hydroxygruppe und der jeweils andere Rest Wasserstoff bedeuten, oder, wenn die Reduktion in $C_{1-4}$-Alkoholen durchgeführt wird, auch zu Verbindungen der allgemeinen Formel I, in welcher X oder Y eine $C_{1-4}$-Alkoxygruppe und der jeweils andere Rest Wasserstoff bedeuten, reduziert, oder Verbindungen der allgemeinen Formel I, in welcher X oder Y eine Hydroxygruppe und der jeweils andere Rest Wasserstoff bedeuten, durch säurekatalysierte Umsetzung mit $C_{1-4}$-Alkoholen zu Verbindungen der allgemeinen Formel I, in welcher X oder Y eine $C_{1-4}$-Alkoxygruppe und der jeweils andere Rest Wasserstoff bedeuten, modifiziert, oder

C) Verbindungen der allgemeinen Formel I, in welcher X, Y, $R^1$ und $R^2$ für Wasserstoff stehen, mit einem oder zwei Äquivalenten einer Verbindung der allgemeinen Formel III,

$$R^{13}\text{-}X^2 \quad \text{(III)}$$

in der $R^{13}$ für eine geradkettige oder verzweigte Alkylgruppe mit 1 bis 18 C-Atomen, eine unsubstituierte oder substituierte Benzylgruppe, eine am Stickstoffatom unsubstituierte oder substituierte Aminoalkylgruppe mit bis zu 12 C-Atomen, wobei die Alkylkette durch weitere $C_{1-4}$-Alkylreste, eine Hydroxygruppe oder eine $C_{1-4}$-Alkoxygruppe substituiert sein kann, einen Alkoxycarbonylalkylrest mit bis zu 6 C-Atomen, einen Halogenalkyl- oder Alkoxyalkylrest mit jeweils bis zu 6 C-Atomen, eine Acylgruppe mit 1 bis 4 C-Atomen und $X^2$ vorzugsweise für Halogen, insbesondere Jod, Brom und Chlor, steht, in Gegenwart von einem oder zwei Äquivalenten einer Base in an sich bekannter Weise an einem oder beiden Indolstickstoffatomen alkyliert oder acyliert, oder indem man bereits eingeführte Reste $R^{13}$ durch übliche Methoden der Organischen Chemie (z.B. Houben-Weyl, Methoden der Organischen Chemie, Georg Thieme Verlag, Stuttgart 1966) zu einem der Reste $R^1$ oder $R^2$ modifiziert, z.B. durch Hydrolyse, Etherspaltung, Amidbildung oder Reduktion, oder

D) Verbindungen der allgemeinen Formel I, in welcher X, Y und einer der Reste $R^1$ und $R^2$ Wasserstoff bedeuten, mit einem Äquivalent einer Verbindung der allgemeinen Formel III in Gegenwart einer Base analog Verfahren C alkyliert oder acyliert und gegebenenfalls den eingeführten Rest $R^{13}$, wie in Verfahren C beschrieben, zu einem der Reste $R^1$ oder $R^2$ modifiziert, oder

E) Verbindungen der allgemeinen Formel I, in der X und Y Wasserstoff bedeuten und $R^1$ und/oder $R^2$ für einen N,N-disubstituierten 3-Amino-2-hydroxy-1-propylrest stehen, durch Alkylierung von Verbindungen der allgemeinen Formel I, in welcher $R^1$ und/oder $R^2$, sowie X und Y für Wasserstoff stehen, mit 1,1-disubstituierten 3-Hydroxyazetidiniumhalogeniden (J. Org. Chem. 1968, 523) herstellt, oder

F) Verbindungen der allgemeinen Formel I, in welcher X und Y Wasserstoff bedeuten und $R^1$ oder $R^2$ für einen 2-Cyanoethylrest oder einen 2-Alkoxycarbonylethylrest mit bis zu 7 C-Atomen steht, durch basenkatalysierte Michael-Addition von Verbindungen der allgemeinen Formel I, in welcher $R^1$ und/oder $R^2$, sowie X und Y für Wasserstoff stehen, an aktivierte Olefine der allgemeinen Formel VIII

$$CH_2=CH-R^{14} \quad (VIII)$$

herstellt, in der $R^{14}$ für eine Cyanogruppe oder einen Alkoxycarbonylrest mit bis zu 5 C-Atomen steht, oder

G) Verbindung der allgemeinen Formel I, in welcher X und Y Wasserstoff bedeuten und $R^1$ oder $R^2$ zusammen einen Propylenrest der allgemeinen Formel IX

```
-CH2-CH-CH2
     |
     R              (IX)
```

bilden, in der R für Hydroxy, $C_{1-4}$-Alkoxy oder Amino steht, durch Umsetzung von Verbindungen der allgemeinen Formel I, in welcher $R^1$ und $R^2$, sowie X und Y für Wasserstoff stehen, mit 2 Äquivalenten einer Base und Epichlorhydrin oder Epibromhydrin herstellt, wobei der zunächst gebildete hydroxysubstituierte Propylenrest nach bekannten Methoden in $C_{1-4}$-alkoxy- oder aminosubstituierte Propylenreste übergeführt werden kann.

Verbindungen der allgemeinen Formel I, in der $R^1$ und/oder $R^2$ für einen Methyl- oder Ethylrest stehen, können auch durch Alkylierung mit Dimethyl- oder Diethylsulfat in bekannter Weise hergestellt werden.

Die beschriebenen Verfahren der Alkylierung oder Acylierung von Verbindungen der allgemeinen Formel I, in welcher $R^1$ und/oder $R^2$, sowie X und Y für Wasserstoff stehen, sind überraschend, da es nicht absehbar war, daß die Einführung von einem oder zwei Alkyl- oder Acylresten selektiv an den Indol-Stickstoffatomen und nicht am Stickstoffatom des Lactamrings erfolgt.

Die Herstellung der Verbindungen der allgemeinen Formel II, in der $R^1$ und $R^2$ Wasserstoff darstellen, erfolgt in Analogie zu den in der Literatur beschriebenen Verfahren (Tetrahedron Lett. 1983, 1441 ; Tetrahedron 1988, 44, 2887 und Angew. Chem. 1980, 92, 463 ; J. Org. Chem. 1987, 1177 ; Tetrahedron Lett. 1987, 4441 und B. Pelcman, Dissertation Stockholm 1988 ; Eur. Pat. Appl. 0269025 und Tetrahedron Lett. 1985, 4015).

Verbindungen der allgemeinen Formel II, in der $R^1$ und $R^2$ gleich oder verschieden sind und Wasserstoff, eine geradkettige oder verzweigte Alkylgruppe mit 1 bis 18 C-Atomen, eine unsubstituierte oder mit bis zu drei $C_{1-4}$-Alkylgruppen, $C_{1-4}$-Alkoxygruppen oder Halogenatomen substituierte Benzylgruppe, oder einen Alkoxyalkylrest mit bis zu 6 C-Atomen bedeuten und $R^3$ bis $R^{10}$ unabhängig von einander Wasserstoff, $C_{1-4}$-Alkyl, $C_{1-4}$-Alkoxy, Halogen, insbesondere Chlor oder Brom, eine Benzyloxygruppe, eine Hydroxygruppe, eine Trifluormethylgruppe oder zwei benachbarte Reste zusammen eine Methylendioxygruppe bedeuten, mit der Maßgabe, daß mindestens einer und bis zu vier der Reste $R^3$ bis $R^{10}$ von Wasserstoff verschieden sind, können in Abwandlung der Synthesen von Arcyriarubinen bzw. von Arcyriaflavinen (vgl. Steglich et al., Angew. Chem. 1980, 92, 463 und Tetrahedron 1988, 44, 2887) wie folgt hergestellt werden (vgl. Syntheseschema 1).

## Syntheseschema 1:

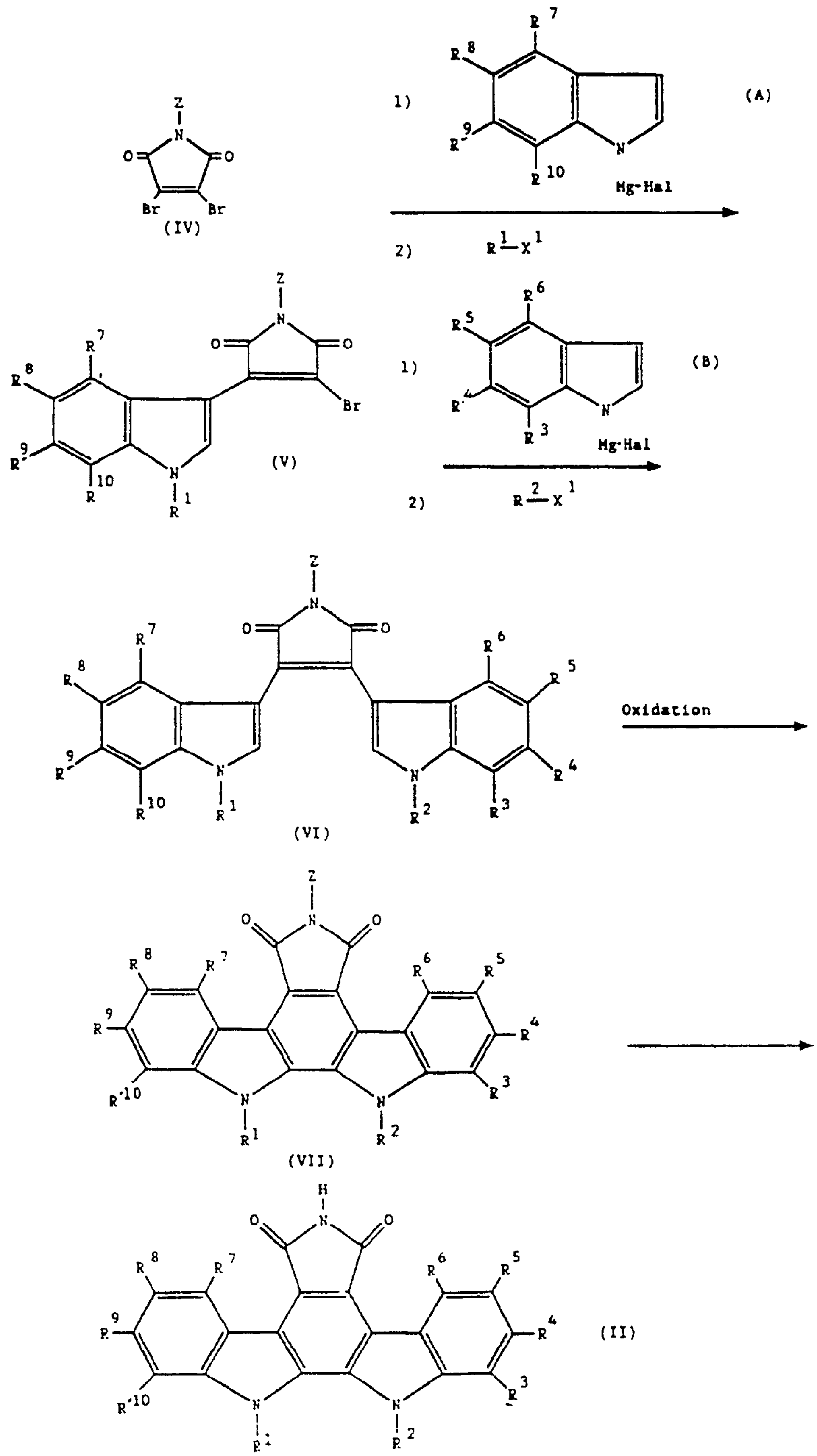

Ein am Stickstoff geeignet substituiertes Dibrommaleinimid (IV) wird mit einem substituierten Indolgrignardreagenz A umgesetzt. Das erhaltene Produkt der allgemeinen Formel V, bei dem $R^1$ Wasserstoff bedeutet, wird gegebenenfalls am Indolstickstoff mit einem Alkylierungsmittel $R^1$-$X^1$, wobei $R^1$ mit Ausnahme von Wasserstoff eine der genannten Bedeutungen hat und $X^1$ für eine leicht austretende Gruppe wie Chlor oder Brom steht, alkyliert, wobei ein Produkt der allgemeinen Formel V erhalten wird, bei dem $R^1$ verschieden von Wasserstoff ist. Erneute Umsetzung von Produkt V mit einem substituierten Indolgrignardreagenz B und gegebenenfalls anschließende Alkylierung mit einem Alkylierungsmittel der allgemeinen Formel $R^2$-$X^1$, in der $R^2$ mit Ausnahme von Wasserstoff eine der genannten Bedeutungen hat und $X^1$ für eine leicht austretende Gruppe wie Chlor oder Brom steht, führt zu einem Produkt der allgemeinen Formel VI. Dieses wird nach Literaturangaben oxidativ cyclisiert, danach wird die substituierte Imidgruppe in Verbindung VII durch geeignete Verfahren in die unsubstituierte Imidgruppe der Verbindung II übergeführt. Bedeutet Z Methyl, so kann das entsprechende N-Methylimid nach Literaturangaben (Tetrahedron 1988, 44, 2887) in das unsubstituierte Imid übergeführt werden.

Bevorzugt sind Verbindungen der allgemeinen Formel I, bei denen $R^1$ und/oder $R^2$ geradkettige oder verzweigte Alkylgruppen mit 1 bis 4 C-Atomen, insbesondere Methyl, Ethyl, n-Propyl, Isopropyl und n-Butyl, 2-Cyanoethylgruppen, Methoxycarbonylmethylgruppen, Benzylgruppen, 2-Methoxyethylgruppen, Acetylgruppen oder auch eine Alkylengruppe mit 2 bis 4 C-Atomen, welche $R^1$ und $R^2$ zusammen bilden, insbesondere die Butylengruppe oder eine hydroxysubstituierte Propylengruppe, oder unsubstituierte oder substituierte Aminoalkylgruppen mit bis zu 12 C-Atomen wie unsubstituierte Aminoalkylgruppen, insbesondere 2-Aminoethyl, 3-Aminopropyl, 1-Amino-2-propyl, N,N-Dialkylaminoalkyl- oder N,N-Alkylbenzyl aminoalkylgruppen mit $C_{1-4}$-Alkylsubstituenten an den Stickstoffatomen und 1-4 C-Atomen in der Alkylkette, wobei die Alkylketten durch weitere $C_{1-4}$-Alkylreste, eine Hydroxygruppe oder eine $C_{1-4}$-Alkoxygruppe substituiert sein können, insbesondere 2-Dimethylaminoethyl-, 3-Dimethylamino-1-propyl-, 3-Dimethylamino-2-propyl-, 2-Diethylaminoethyl-, 2-[N-Benzyl-N-methylamino]ethyl-, 3-[N-Benzyl-N-methylamino]propyl-, 3-Diethylamino-2-hydroxy-1-propyl-, 3-Diethylamino-2-methoxy-1-propyl-, 3-Dimethylamino-2-methoxy-1-propyl-, 3-Dimethylamino-2-hydroxy-1-propyl-, oder 2-Piperidinoethyl-, 3-Piperidinopropyl-, 2-Pyrrolidinoethyl-, 3-Pyrrolidinopropyl-, 2-Morpholinoethyl-, 3-Morpholinopropyl-, 3-Morpholino-2-hydroxy-1-propyl-, Pyrrolidin-2-ylmethyl- und N-Methylpyrrolidin-2-ylmethylgruppen bedeuten und einer oder zwei der Reste $R^3$ bis $R^{10}$ von Wasserstoff verschieden sind und unabhängig voneinander für Methyl, Ethyl, Chlor, Brom, Methoxy, Hydroxy oder unsubstituierte oder substituierte Aminoalkoxygruppen mit bis zu 12 C-Atomen stehen, wobei die Aminoalkylreste denen entsprechen, die für $R^1$ und/oder $R^2$ als besonders geeignet definiert werden.

Bevorzugt sind auch Indolocarbazol-Derivate der allgemeinen Formel I, in denen $R^1$ und/oder $R^2$ Wasserstoff, Methyl-, Ethyl-, n-Propyl-, Isopropyl-, n-Butyl-, Cyanomethyl-, 2-Cyanoethyl-, Benzyl-, Acetyl-, Methoxycarbonylmethyl-, 2-Methoxyethyl-, 2-Aminoethyl-, 3-Aminopropyl-, 1-Amino-2-propyl-, 2-Dimethylaminoethyl, 3-Dimethylamino-1-propyl-, 3-Dimethylamino-2-propyl-, 2-Diethylaminoethyl-, 2-[N-Benzyl-N-methylamino]ethyl-, 3-[N-Benzyl-N-methylamino]propyl-, 3-Dimethylamino-2-hydroxy-1-propyl-, 3-Diethylamino-2-hydroxy-1-propyl-, 3-Diethylamino-2-methoxy-1-propyl-, 3-Piperidinopropyl-, 2-Piperidinoethyl-, 2-Pyrrolidinoethyl-, 3-Pyrrolidinopropyl-, 2-Morpholino-ethyl, 3-Morpholinopropyl-, 3-Morpholino-2-hydroxy-1-propyl-, Pyrrolidin-2-ylmethyl- oder N-Methyl-pyrrolidin-2-ylmethylgruppen oder $R^1$ und $R^2$ gemeinsam eine Butylen- oder eine hydroxysubstituierte Propylengruppe, $R^3$ bis $R^{10}$ Wasserstoff, Chlor, Brom oder Hydroxy-, Methyl-, Ethyl-, Methoxy-, 2-Aminoethoxy-, 3-Aminopropoxy-, 1-Amino-2-propoxy-, 2-Dimethylaminoethoxy-, 3-Dimethylamino-1-propoxy, 3-Dimethylamino-2-propoxy-, 2-Diethylaminoethoxy-, 2-[N-Benzyl-N-methylamino]ethoxy-, 3-[N-Benzyl-N-methyl-amino]propoxy-, 3-Dimethylamino-2-hydroxy-1-propoxy-, 2-Piperidinoethoxy-, 3-Piperidinopropoxy-, 2-Pyrrolidino-ethoxy-, 3-Pyrrolidinopropoxy-, 2-Morpholinoethoxy-, 3-Morpholinopropoxy-, Pyrrolidin-2-ylmethoxy- oder N-Methyl-pyrrolidin-2-ylmethoxygruppen und X und Y entweder beide jeweils Wasserstoff oder ein Rest X oder Y Wasserstoff und der andere Rest eine Hydroxy-, Methoxy-, Ethoxy-, Propoxy-, Isopropoxy- oder n-Butoxygruppe darstellt.

Besonders bevorzugt sind Verbindungen der allgemeinen Formel I, bei denen $R^5$ oder $R^8$ für Methoxy, Methyl oder Chlor oder bei denen $R^4$ oder $R^9$ für Methoxy, Methyl oder Chlor oder bei denen $R^5$ und $R^8$ gleich sind und für Methoxy, Hydroxy, Methyl, Brom oder Chlor oder bei denen $R^4$ und $R^9$ gleich sind und für Methoxy, Methyl, Brom oder Chlor oder bei denen $R^3$ und $R^{10}$ gleich sind und für Methyl, Brom oder Chlor oder bei denen $R^4$ und $R^5$ oder $R^8$ und $R^9$ gleich sind und für Methoxy oder bei denen $R^4$, $R^5$, $R^8$ und $R^9$ gleich sind und für Methoxy stehen und die anderen der Reste $R^3$ bis $R^{10}$ jeweils Wasserstoff bedeuten.

Zur Durchführung der Reduktionen nach Verfahren A wird vor zugsweise Zinkamalgam/Chlorwasserstoffgas in C1-C4-Alkoholen, Zinkamalgam in Eisessig oder Zink in Eisessig eingesetzt. Um zur Stufe des Lactams zu reduzieren, wird vorzugsweise bei erhöhter Temperatur von ca. 50°C bis 150°C gearbeitet. Diese Reduktion ist an vergleichbaren Indolocarbazolen bisher nur für ein Derivat beschrieben, das am Imidstickstoffatom durch eine Benzylgruppe geschützt ist (J. Org. Chem. 1987, 1177 und Tetrahedron Lett. 1983, 1441).

Zur Durchführung der Reduktionen nach Verfahren B wird vorzugsweise Zinkamalgam/Chlorwasserstoffgas in C1-C4-Alkoholen bei Temperaturen unter 20°C oder Borhydride, wie z.B. Natriumborhydrid, vorzugsweise in C1-C4-Alkoholen oder Alkohol/Wasser-Gemischen, oder Lithiumaluminiumhydrid in einem aprotischen Lösungsmittel eingesetzt. Verbindungen der allgemeinen Formel I, in welcher X oder Y eine $C_{1-4}$-Alkoxygruppe und der jeweils andere Rest Wasserstoff bedeuten, werden z.B. erhalten, wenn die Reduktion mit Zinkamalgam/Chlorwasserstoffgas in C1-4-Alkoholen bei 0°C durchgeführt wird.

Falls bei der Reduktion mit Zinkamalgam/Chlorwasserstoffgas in C1-C4-Alkoholen Gemische von Verbindungen der allgemeinen Formel I anfallen, bei denen X oder Y entweder eine Hydroxy- oder eine C1-C4-Alkoxygruppe und der andere der beiden Reste Wasserstoff bedeuten, können diese durch übliche Verfahren wie Kristallisation oder Chromatographie getrennt werden.

Verbindungen der allgemeinen Formel I, bei denen X oder Y eine Hydroxy- oder eine C1-C4-Alkoxygruppe und $R^1$ und/oder $R^2$ Wasserstoff bedeuten, können anschließend gegebenenfalls nach den Verfahren C bis G zu Verbindungen der allgemeinen Formel I, bei denen X oder Y eine Hydroxy- oder eine C1-C4-Alkoxygruppe und der andere der beiden Reste Wasserstoff und $R^1$ und $R^2$ eine der oben angegebenen Definitionen, jedoch nicht beide gleichzeitig Wasserstoff, bedeuten, alkyliert oder acyliert werden.

Zur Durchführung der Alkylierungen oder Acylierungen nach Verfahren C oder D mit Verbindungen III oder nach Verfahren E und G besonders geeignete Basen sind Hydride, Carbonate, Hydroxide, Oxide oder Alkoxide der Alkali- oder Erdalkalimetalle oder lithiumorganische Verbindungen.

Eine zur Durchführung der Michael-Addition nach Verfahren F besonders geeignete Base ist 1,8-Diazabicyclo[5,4o]undec-7-en (DBU).

Verbindungen der allgemeinen Formel I nach Verfahren A bis F, die als Regioisomerengemische anfallen, können in Form der Regioisomerengemische verwendet werden. Gegebenenfalls können die Regioisomeren durch bekannte Trennverfahren wie Kristallisation oder Chromatographie getrennt werden.

Verbindungen der allgemeinen Formel I, bei denen einer oder bis zu vier der Reste $R^3$ bis $R^{10}$ für eine Hydroxygruppe stehen, können auch in an sich bekannter Weise durch Etherspaltung von Verbindungen der allgemeinen Formel I, bei denen einer oder mehrere der Reste $R^3$ bis $R^{10}$ für eine C1-C4-Alkoxygruppe stehen, hergestellt werden.

Verbindungen der allgemeinen Formel I, bei denen einer oder bis zu vier der Reste $R^3$ bis $R^{10}$ eine unsubstituierte oder substituierte Aminoalkoxygruppe stehen, können auch in an sich bekannter Weise durch Aminoalkylierung von Verbindungen der allgemeinen Formel I, bei denen einer oder bis zu vier der Reste $R^3$ bis $R^{10}$ für eine Hydroxygruppe stehen, hergestellt werden.

Verbindungen der allgemeinen Formel I, bei denen X und Y Wasserstoff bedeuten und einer oder zwei der Reste $R^3$ bis $R^{10}$ für $C_{1-4}$-Alkyl, $C_{1-4}$-Acyl, Chlor, Brom oder Nitro stehen, können auch nach bekannten Methoden der elektrophilen aromatischen Substitution aus Verbindungen der allgemeinen Formel I, bei denen X und Y Wasserstoff bedeuten und der eine oder die beiden entsprechenden Reste $R^3$ bis $R^{10}$ für Wasserstoff stehen, hergestellt werden.

Verbindungen der allgemeinen Formel I, die ein chirales Zentrum aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Basische Verbindungen der allgemeinen Formel I, welche ein basisches Zentrum an mindestens einem der Reste $R^1$ bis $R^{10}$ aufweisen, werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen sind potente Inhibitoren von Proteinkinasen wie der Proteinkinase C. So zeigt z.B. die Verbindung von Beispiel 3.a im Enzym-Assay der mit Phosphatidylserin und Diacylglycerol aktivierten Proteinkinase C eine 50%ige Inhibierung bei einer Konzentration von 0,31 µmol/Liter. Der Versuch wurde gemäß EP-OS-0255126 (Hemmung von Proteinkinase C) durchgeführt. Es sind zwar schon Indolocarbazole als Inhibitoren der Proteinkinase C beschrieben worden (J. Antibiot. 1977, 30, 275 ; Biochem Biophys. Res. Commun. 1986, 135, 397). Dabei handelt es sich aber hauptsächlich um Indolocarbazol-N,N-glykoside mikrobiellen oder semisynthetischen Ursprungs.

Die Proteinkinase C spielt für die intracelluläre Signaltransduktion eine wichtige Schlüsselrolle und ist eng

mit der Regulation von kontraktilen, sekretorischen und proliferativen Prozessen verknüpft. Aufgrund dieser Eigenschaften können die erfindungsgemäßen Verbindungen zur Behandlung von Herz- und Gefäßkrankheiten wie Thrombosen, Arteriosklerose, Hypertension, von Entzündungsprozessen, Allergien, Krebs, viralen Erkrankungen und bestimmten degenerativen Schäden des Zentralnervensystems verwendet werden. Die Verbindungen können in der jeweils geeigneten Formulierung enteral oder parenteral in Dosen von 1 bis 500 mg/kg, bevorzugt 1 bis 50 mg/kg verabreicht werden.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung. Feste Trägerstoffe sind z.B. Särke, Lactose, Mannit, Methylcellulose, Talkum, hochdisperse Kieselsäuren, höhermolekulare Fettsäuren (wie Stearinsäure), Gelatine, Agar-Agar, Calciumphosphat Magnesiumstearat, tierische und pflanzliche Fette, feste hochmolekulare Polymere (wie Polyäthylenglykol) ; für orale Applikation geeignete Zubereitungen können gewünschtenfalls zusätzliche Geschmacks- und/oder Süßstoffe enthalten.

Die folgenden Beispiele dienen der näheren Erläuterung der Erfindung :

**Beispiel 1** (nach Verfahren A)

3,9-Dichlor-6,7,12,13-tetrahydro-5-oxo-5H-indolo[2,3-a]pyrrolo[3,4-c]carbazol

2,5 (38 mmol) Zinkpulver und 0,25 g (0,9 mmol) Quecksilber(II)chlorid in 3 ml Wasser werden 20 min. bei 20°C gerührt, dann 2 Tropfen konz. Salzsäure zugefügt und 1 min. weitergerührt. Sofort danach wird das Zinkpulver zunächst mit Wasser, dann mit Ethanol und zum Schluß mit getrocknetem Ethanol gewaschen. Das Zinkpulver wird in 50 ml trockenem Ethanol suspendiert. Unter Rühren werden 0,465 g (1,18 mmol) 3,9-Dichlor-6,7,12,13-tetrahydro-5,7-dioxo-5H-indolo[2,3-a]pyrrolo[3,4-c]carbazol zugefügt und es wird bei langsamem Durchleiten von getrocknetem Chlorwasserstoff 2 h unter Rückfluß erhitzt. Nach dem Abkühlen wird eingedampft und der Rückstand zwischen Kaliumcarbonatlösung (150 ml) und Ethylacetat (300 ml) verteilt. Die organische Phase wird abgetrennt, über Natriumsulfat getrocknet und eingedampft. Der Rückstand wird mit Diisopropylether/Ethylacetat heiß verrührt und nach dem Abkühlen die Kristalle abfiltriert. Man erhält 3,9-Dichlor-6,7,12,13-tetrahydro-5-oxo-5H-indolo[2,3-a]pyrrolo[3,4-c]carbazol in Form blaßbeiger Kristalle, die sich > 300°C zersetzen.

Das als Ausgangsprodukt verwendete 3,9-Dichlor-6,7,12,13-tetrahydro-5,7-dioxo-5H-indolo[2,3-a]pyrrolo[3,4-c]carbazol wird wie folgt hergestellt :
1,19 g (3 mmol) 3,9-Dichlor-4c,6,7,7a,12,13-hexahydro-5,7-dioxo-5H-indolo[2,3-a]pyrrolo[3,4-c]carbazol und 0,70 g (3,1 mmol) DDQ in 100 ml Ethylacetat werden 16 h unter Rückfluß gekocht. Nach dem Abkühlen werden die Kristalle abfiltriert. Man erhält 3,9-Dichlor-6,7,12,13-tetrahydro-5,7-dioxo-5H-indolo[2,3-a]pyrrolo[3,4-c]carbazol in Form gelber Kristalle, die sich > 300°C zersetzen.

Das als Ausgangsprodukt verwendete 3,9-Dichlor-4c,6,7,7a,12,13-hexahydro-5,7-dioxo-5H-indolo[2,3-a]pyrrolo[3,4]-carbazol wird wie folgt hergestellt :
8,1 g (18,8 mmol) 5-[4-Chlorphenyl)azo]-6-[N'-(4-chlorphenyl)hydrazino]-1,3,3a,4,7,7a-hexahydro-1,3-dioxoisoindol werden mit Polyphosphorsäuretrimethylsilylester (PPSE), der nach Literaturangaben (Bull. Chem. Soc. Jpn., 1986, 59, 2171) aus 18 g (127 mmol) Phosphorpentoxid und 50 ml (235 mmol) Hexamethyldisiloxan in 100 ml Dichlormethan frisch bereitet wird, in 100 ml Nitromethan unter Argonatmosphäre 24 h unter Rückfluß gekocht. Nach dem Abkühlen werden 100 ml Wasser unter Rühren zugetropft und das ausgefallene Rohprodukt abfiltriert. Anschließend wird an Kieselgel mit Toluol/Ethylacetat 1 : 1 chromatographiert, die Fraktion mit dem $R_f$ 0.5 isoliert, mit Dichlormethan/Methanol verrührt und die Kristalle abfiltriert. Man erhält 3,9-Dichlor-4c,6,7,7a,12,13-hexahydro-5,7-dioxo-5H-indolo[2,3-a]pyrrolo[3,4-c]carbazol in Form gel ber Kristalle vom Schmp. > 300°C.

Das als Ausgangsprodukt verwendete 5-[(4-Chlorphenyl)azo]-6-[N'-(4-chlorphenyl)hydrazino]-1,3,3a,4,7,7a-hexahydro-1,3-dioxo-isoindol wird wie folgt hergestellt :
8 g (24,4 mmol) 1,3,3a,4,7,7a-Hexahydro-1,3-dioxo-5,6-bis(trimethylsiloxy)-isoindol und 11,4 g (80 mmol) 4-Chlorphenylhydrazin werden in 50 ml Methanol und 50 ml Eisessig 16 h unter Rückfluß gekocht. Die nach dem Abkühlen ausgefallenen Kristalle werden abfiltriert, mit Methanol mehrfach nachgewaschen und getrocknet. Man erhält 5-[(4-Chlorphenyl)azo]-6-[N'-(4-chlorphenyl)hydrazino]-1,3,3a,4,7,7a-hexahydro-1,3-dioxo-isoindol in Form gelbgrüner Kristalle.

Das als Ausgangsprodukt verwendete 1,3,3a,4,7,7a-Hexahydro-1,3-dioxo-5,6-bis(trimethylsiloxy)-isoindol

wird nach B. Pelcman (Dissertation Stockholm 1988, The Royal Institute of Technology, Department of Organic Chemistry) wie folgt hergestellt:

75,2 g (0,326 mol) 2,3-Bis(trimethylsiloxy)-1,3-butadien (Chem. Lett. 1977, 1219) und 31,6 g (0,326 mol) Maleinsäureimid werden in 1 l Toluol 16 h unter Rückfluß gekocht. Nach dem Abkühlen wird filtriert, das Filtrat im Vakuum eingedampft und der Rückstand aus 600 ml Cyclohexan kristallisiert. Man erhält 1,3,3a,4,7,7a-Hexahydro-1,3-dioxo-5,6-bis(trimethylsiloxy)-isoindol in Form farbloser Kristalle.

In analoger Weise werden die folgenden Verbindungen erhalten:

6,7,12,13-Tetrahydro-3,9-dimethoxy-5-oxo-5H-indolo[2,3-a]pyrrolo[3,4-c]carbazol (1.a)
Schmp. > 300°C aus Ethylacetat

6,7,12,13-Tetrahydro-3,9-dimethyl-5-oxo-5H-indolo[2,3-a]pyrrolo[3,4-c]carbazol (1.b)
Schmp. > 300°C (Zers.) aus Diisopropylether/Ethylacetat

3,9-Dibrom-6,7,12,13-tetrahydro-5-oxo-5H-indolo[2,3-a]pyrrolo[3,4-c]carbazol (1.c)
Schmp. > 300°C (Zers.) aus Diisopropylether/Ethylacetat

2,10-Dichlor-6,7,12,13-tetrahydro-5-oxo-5H-indolo[2,3-a]pyrrolo[3,4-c]carbazol (1.d)

1,11-Dichlor-6,7,12,13-tetrahydro-5-oxo-5H-indolo[2,3-a]pyrrolo[3,4-c]carbazol (1.e)
Schmp. > 340°C aus Ethanol

**Beispiel 2** (nach Verfahren A)

6,7,12,13-Tetrahydro-9-methoxy-5-oxo-5H-indolo[2,3-a]-pyrrolo[3,4-c]carbazol und
6,7,12,13-Tetrahydro-3-methoxy-5-oxo-5H-indolo[2,3-a]-pyrrolo[3,4-c]carbazol

265 mg (0,75 mmol) 6,7,12,13-Tetrahydro-3-methoxy-5,7-dioxo-5H-indolo[2,3-a]pyrrolo[3,4-c]carbazol und 2,0 g Zinkamalgam werden in 35 ml Ethanol unter kräftigem Rühren und unter Einleiten von HCl-Gas 1 h zum Sieden erhitzt. Man läßt abkühlen, rotiert das Lösungsmittel ab und behandelt den Rückstand 5 mal mit jeweils 100 ml heißem Essigester. Die vereinigten Essigesterextrakte werden mit verdünnter wäßriger Natriumcarbonatlösung ausgewaschen, über Natriumsulfat getrocknet und eingeengt. Der Rückstand wird zunächst mit heißem Aceton, dann mit heißem Acetonitril behandelt und abgesaugt.

Man erhält ein ca. 3 : 5-Regioisomerengemisch von 6,7,12,13 Tetrahydro-9-methoxy-5-oxo-5H-indolo[2,3-a]pyrrolo [3,4-c]carbazol und 6,7,12,13-Tetrahydro-3-methoxy-5-oxo-5H-indolo[2,3-a]pyrrolo[3,4-c]carbazol in Form hellbeiger Kristalle vom Schmelzpunkt 313°C. Dünnschichtchromatographie auf Kieselgel 60 $F_{254}$, Laufmittel Ethylacetat, $R_f$ = 0.33.

Das als Ausgangsmaterial verwendete 6,7,12,13-Tetrahydro 3-methoxy-5,7-dioxo-5H-indolo[2,3-a]pyrrolo[3,4-c]carbazol wird wie folgt hergestellt:

1,0 g (2,3 mmol)3-(3-Methoxy-6-nitro)phenyl-6-(2-nitrophenyl)phthalimid, 3,0 g (11,6 mmol) Triphenylphosphin und 50 ml Collidin werden 30 h unter Rückfluß erhitzt. Das Collidin wird im Vakuum bei 0,1 Torr abdestilliert und der Rückstand mit 30 ml Methanol verrührt und über Nacht im Kühlschrank aufbewahrt. Der ausgefallene Niederschlag wird abgesaugt, das Filtrat eingeengt. Der ölige Rückstand wird in wenig Chloroform aufgenommen und an Kieselgel flaschchromatographiert. Es wird zunächst mit Chloroform und dann mit Methanol eluiert. Man erhält ein dunkelgelbes Produkt von Schmp. > 330°C (Zers.). Dünnschichtchromatographie auf Kieselgel 60 $F_{254}$, Laufmittel Hexan/Ethylacetat 1 : 1, $R_f$ 0.23.

Das als Ausgangsmaterial verwendete 3-(3-Methoxy-6-nitrophenyl)-6-(2-nitrophenyl)-phthalimid wird wie folgt her gestellt.

1,0 g (2,3 mmol) 3-(3-Methoxy-6-nitrophenyl)-6-(2-nitrophenyl)-2a,3,6,6a-tetrahydro-phthalimid und 2,2 g (9,8 mmol) 2,3-Dichlor-5,6-dicyano-p-benzochinon werden in 25 ml tert. Butylbenzol 5 h unter Rückfluß erhitzt. Man läßt abkühlen, saugt den Niederschlag ab und behandelt ihn 5 min mit ca. 20 ml Methanol im Ultraschallbad. Nach dem Absaugen erhält man bräunliche Kristalle vom Schmp. 280-281°C.

Das als Ausgangsprodukt verwendete 2a,3,6,6a-Tetrahydro-3-(3-methoxy-6-nitrophenyl)-6-(2-nitrophenyl)-phthalimid wird wie folgt hergestellt:

3,0 g (9,2 mmol) (E,E)-1-(3-Methoxy-6-nitrophenyl)-4-(2-nitrophenyl)-1,3-butadien und 1,0 g (10 mmol) Maleinimid werden in 30 ml Toluol 24 h unter Rühren auf 145°C erhitzt. Man läßt abkühlen, saugt den Niederschlag ab und behandelt ihn 5 min im Ultraschallbad mit ca. 20 ml Methylenchlorid. Man erhält ein bräunliches Produkt, Schmp. 217-218°C.

Das als Ausgangsprodukt verwendete (E,E)-1-(3-Methoxy-6-nitrophenyl)-4-(2-nitrophenyl)-1,3-butadien wird wie folgt hergestellt:

21,5 g (42,3 mmol) 3-Methoxy-6-nitrobenzyl-triphenylphosphoniumbromid und 2,5 g 18-Krone-6 werden in 350 ml trockenem Methylenchlorid gelöst und unter Stickstoff auf 0°C abgekühlt. Innerhalb einer halben Stunde versetzt man portionsweise mit 5,8 g (42,3 mmol) trockenem, fein pulverisiertem Kaliumcarbonat und rührt danach

noch eine halbe Stunde bei 0°C. Die Reaktionsmischung verfärbt sich von ockergelb nach tiefviolett. Man gibt 7,1 g (40,2 mmol) o-Nitrozimtaldehyd hinzu und rührt 2 1/2 Tage unter Stickstoff bei Raumtemperatur weiter. Die Reaktionsmischung wird mit 200 ml Wasser gut ausgewaschen und die organische Phase über Natriumsulfat getrocknet. Nach dem Trocknen wird das Methylenchlorid abrotiert und der dunkle Rückstand mit wenig Toluol im Ultraschallbad behandelt. Der Niederschlag wird abgesaugt und das Filtrat verworfen. Der Niederschlag wird erneut zunächst mit wenig Methanol und dann nochmals mit Toluol im Ultraschallbad behandelt. Nach dem Absaugen erhält man reines E,E-Dien vom Fp. 162-163°C. Im Toluolfiltrat befindet sich eine Mischung aus E,Z- und E,E-Dien. Zugabe von 3 Tropfen einer ges. Jodlösung in Toluol und Rühren über Nacht bei Raumtemperatur führt zur Bildung des E,E-Produkts.

Das als Ausgangsprodukt verwendete 3-Methoxy-6-nitro benzyl-triphenylphosphoniumbromid wird wie folgt hergestellt :

Eine Suspension von 11,6 g (65 mmol) N-Bromsuccinimid und 10,9 g (65 mmol) 3-Methyl-4-nitroanisol in 80 ml Tetrachlorkohlenstoff wird ca. 4 h unter Rückfluß erhitzt. Hierbei wird die Reaktionsmischung mit einer 500 Watt Photolampe bestrahlt und halbstündlich mit jeweils 0,1 g Dibenzoylperoxid versetzt. Nach dem Abkühlen wird die dunkle Reaktionsmischung filtriert und das Filtrat eingeengt. Der ölige Rückstand wird in 150 ml trockenem Dimethylformamid gelöst und nach Zugabe von 15,3 g (58,4 mmol) Triphenylphosphin 4 h auf 100°C erwärmt. Nach dem Abkühlen wird das Lösungsmittel im Vakuum abgezogen und der ölige Rückstand mit wenig Aceton verrieben. Der ausgefallene gelbliche Niederschlag wird abgesaugt, mit wenig kaltem Aceton ausgewaschen und an der Luft getrocknet. Fp. 230-233°C.

In analoger Weise werden die folgenden Verbindungen als Regioisomerenmischung erhalten :
2-Chlor-6,7,12,13-tetrahydro-5-oxo-5H-indolo[2,3-a]-pyrrolo[3,4-c]carbazol und 10-Chlor-6,7,12,13-tetrahydro-5-oxo-5H-indolo[2,3-a]-pyrrolo[3,4-c]carbazol (2.a)
Schmp. > 300°C (Zers.) aus Methanol

<u>**Beispiel 3** (nach Verfahren C)</u>

2(und 10)-chlor-12-ethyl-6,7,12,13-tetrahydro-5-oxo-5H-indolo[2,3-a]pyrrolo[3,4-c]carbazol (3.a) und 2(und 10)-Chlor-12,13-diethyl-6,7,12,13-tetrahydro-5-oxo 5H-indolo[2,3-a]pyrrolo[3,4-c]carbazol (3.b) und 2(und 10)-Chlor-13-ethyl-6,7,12,13-tetrahydro-5-oxo-5H-indolo[2,3-a]pyrrolo[3,4-c]carbazazol (3.c)

Unter Stickstoff versetzt man eine Suspension von 13,3 mg (0,44 mmol) Natriumhydrid (80%ig in Paraffinöl) in 15 ml trockenem Dimethylformamid portionsweise mit 75 mg (0,21 mmol) 2(und 10)-Chlor-6,7,12,13-tetrahydro-5-oxo-5H-indolo[2,3-a]pyrrolo[3,4-c]carbazol (Beispiel 2.a) und rührt 30 min bei Raumtemperatur. Danach tropft man eine Lösung von 40,4 mg (0,37 mmol) Ethylbromid in 1 ml Dimethylformamid hinzu und rührt 20 h bei Raumtemperatur weiter. Das Dimethylformamid wird abrotiert und der Rückstand an Kieselgel 60 flashchromatographiert. Als Elutionsmittel dient eine Mischung aus Chloroform/Methanol 30 : 1.

Man erhält als zuerst eluiertes Produkt 2(und 10)-Chlor-12,13-diethyl-6,7,12,13-tetrahydro-5-oxo-5H-indolo-[2,3-a]pyrrolo[3,4-c]carbazol in Form hellgelber Kristalle, Schmp. 263°C. Dünnschichtchromatographie auf Kieselgel 60 $F_{254}$, Laufmittel Toluol/Ethanol 10 : 2, $R_f$ 0.50.

Als zweites Produkt wird 2(und10)-Chlor-13-ethyl-6,7,12,13-tetrahydro-5-oxo-5H-indolo[2,3-a]-pyrrolo[3,4-c] carbazol eluiert. Es bildet dunkelgelbe Kristalle. Schmp. > 360°C. Dünnschichtchromatographie auf Kieselgel 60 $F_{254}$, Laufmittel Toluol/Ethanol 10 : 2, $R_f$ 0.45.

Als zuletzt eluiertes Produkt wird 2(und10)-Chlor-12-ethyl-6,7,12,13-tetrahydro-5-oxo-5H-indolo[2,3-a]-pyrrolo[3,4-c]carbazol in Form beiger Kristalle erhalten. Schmp. 245°C (Zers.). Dünnschichtchromatographie auf Kieselgel 60, Laufmittel Toluol/Ethanol 10 : 2, $R_f$ 0.43.

<u>**Beispiel 4** (nach Verfahren C)</u>

12-Ethyl-6,7,12,13-tetrahydro-3,9-dimethoxy-5-oxo-5H-indolo[2,3-a]pyrrolo[3,4-c]carbazol und
13-Ethyl-6,7,12,13-tetrahydro-3,9-dimethoxy-5-oxo-5H-indolo[2,3-a]-pyrrolo[3,4-c]carbazol

15 mg (0,50 mmol) Natriumhydrid (80%ig in Mineralöl) werden in 20 ml trockenem Dimethylformamid suspendiert und 150 mg (0,40 mmol) 6,7,12,13-Tetrahydro-3,9-dimethoxy-5-oxo-5H-indolo[2,3-a]pyrrolo[3,4-c]carbazol (Beispiel 1.a) portionsweise bei Raumtemperatur zugegeben. Nach Abklingen der Gasentwicklung wird 1 h bei Raumtemperatur nachgerührt, dann werden 75 mg (0,48 mmol) Ethyljodid zugefügt und 16 h bei Raumtemperatur gerührt. Das Lösungsmittel wird im Vakuum abdestilliert und der Rückstand an Kieselgel mit Dichlormethan/Methanol 95 : 5 chromatographiert. Die Fraktion mit dem $R_f$ 0.3 wird isoliert, mit Diisopropylether/Ethylacetat verrührt und die gebildeten Kristalle abfiltriert.

Man erhält ein ca. 3 : 1-Regioisomerengemisch von 12-Ethyl-6,7,12,13-tetrahydro-3,9-dimethoxy-5-oxo-5H-indolo[2,3-a]pyrrolo[3,4-c]carbazol und 13-Ethyl-6,7,12,13-tetrahydro-3,9-dimethoxy-5-oxo-5H-indolo[2,3-a]pyrrolo[3,4-c]carbazol in Form beiger Kristalle, die sich oberhalb 280°C zersetzen.

In analoger Weise erhält man :
12-Methyl-6,7,12,13-tetrahydro-3,9-dimethyl-5-oxo-5H-indolo[2,3-a]pyrrolo[3,4-c]carbazol und 13-Methyl-6,7,12,13-tetrahydro-3,9-dimethyl-5-oxo-5H-indolo[2,3-a]pyrrolo[3,4-c]carbazol (4.a)
Schmp. > 340°C aus Diisopropylether/Ethylacetat 93 : 7 — Regioisomerengemisch

**Beispiel 5** (nach Verfahren C)

12,13-Diethyl-6,7,12,13-tetrahydro-3,9-dimethoxy-5-oxo-5H-indolo[2,3-a]pyrrolo[3,4-c]carbazol

Das nach Umsetzung von 150 mg (0,40 mmol) 6,7,12,13-Tetrahydro-3,9-dimethoxy-5-oxo-5H-indolo[2,3-a]pyrrolo[3,4-c]-carbazol (Beispiel 1.a) mit 30 mg (1 mmol) Natriumhydrid (80%ig in Mineralöl) und 150 mg (0,96 mmol) Ethyljodid analog Beispiel 4 erhaltene Rohprodukt wird chromatographisch an Kieselgel mit Dichlormethan/Methanol 95 : 5 getrennt. Die Fraktion mit dem Rf 0.35 wird isoliert, mit Diisopropylether/Ethylacetat verrührt und die gebildeten Kristalle abfiltriert. Man erhält 12,13-Diethyl-6,7,12,13-tetrahydro-3,9-dimethoxy-5-oxo-5H-indolo[2,3-a]pyrrolo[3,4-c]carbazol in Form beiger Kristalle vom Schmp. 193-196°C.

In analoger Weise erhält man :
12,13-Dimethyl-6,7,12,13-tetrahydro-3,9-dimethyl-5-oxo-5H-indolo[2,3-a]pyrrolo[3,4-c]carbazol (5.a)
Schmp. > 280°C (Zers.) aus Diisopropylether/Ethylacetat
12,13-Dimethyl-6,7,12,13-tetrahydro-3,9-dimethoxy-5-oxo-5H-indolo[2,3-a]pyrrolo[3,4-c]carbazol (5.b)

Beispiel 6

6,7,12,13-Tetrahydro-3,9-dihydroxy-5-oxo-5H-indolo[2,3-a]-pyrrolo[3,4-c]carbazol

250 mg (0,67 mmol) 6,7,12,13-Tetrahydro-3,9-dimethoxy-5-oxo-5H-indolo[2,3-a]-pyrrolo[3,4-c]carbazol (Beispiel 1.a) in 10 ml trockenem Dichlormethan werden bei −10°C mit einer Lösung von 506 mg (2 mmol) Bortribromid in 5 ml Dichlormethan versetzt. Es wird 1 Stunde bei −10°C, dann 2 Stunden bei 20°C gerührt. Anschließend wird unter Eiskühlung Wasser zugetropft (50 ml) und mit Ethylacetat (300 ml) extrahiert. Das nicht gelöste Rohprodukt wird abfiltriert und zusammen mit dem Rohprodukt, das durch Eindampfen der organischen Phase erhalten wird, an Kieselgel mit Dichlormethan/Methanol 9 : 1 chromatographisch getrennt. Die Fraktion mit dem Rf 0,1 wird isoliert, mit Diisopropylether/Methanol verrührt und die gebildeten Kristalle abfiltriert.

Man erhält 6,7,12,13-Tetrahydro-3,9-dihydroxy-5-oxo-5H-indolo[2,3-a]-pyrrolo[3,4-c]carbazol in Form beiger Kristalle von Schmp. > 330°C.

## Patentansprüche

1. Indolocarbazol-Derivate der allgemeinen Formel I

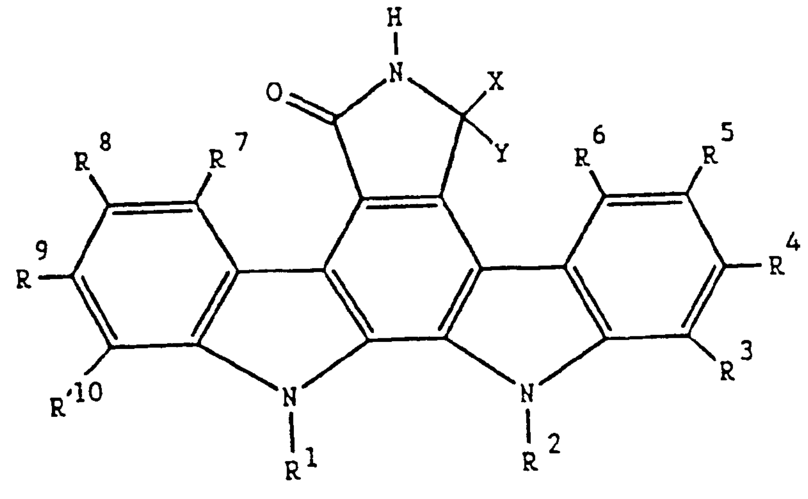

(I)

in welcher $R^1$ und $R^2$ gleich oder verschieden sind und Wasserstoff, eine geradkettige oder verzweigte Alkylgruppe mit 1 bis 18 C-Atomen, eine Cyanoalkylgruppe mit 2 bis 4 C-Atomen, eine unsubstituierte oder mit bis zu drei $C_{1-4}$-Alkylgruppen, $C_{1-4}$-Alkoxygruppen oder Halogenatomen substituierte Benzylgruppe, eine Aminoalkylgruppe mit bis zu 12 C-Atomen, am Stickstoffatom unsubstituiert oder mono- oder disubstituiert durch Benzyl oder Alkylreste mit 1 bis 4 C-Atomen oder bei der zwei Substituenten am Stickstoffatom zusammen mit dem Stickstoffatom oder ein Substituent am Stickstoffatom und ein Substituent der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, wobei die Alkylkette durch weitere $C_{1-4}$-Alkylreste, eine Hydroxygruppe oder eine $C_{1-4}$-Alkoxygruppe substituiert sein kann, einen Alkoxycarbonylalkylrest mit bis zu 7 C-Atomen, einen Rest—$CH_2$-CO-$NR^{11}R^{12}$, bei dem $R^{11}$ und $R^{12}$ gleich oder verschieden sind und für Wasserstoff, eine Alkylgruppe mit 1 bis 6 C-Atomen oder eine Benzylgruppe stehen, einen Halogenalkyl-, Hydroxyalkyl- oder Alkoxyalkylrest mit jeweils bis zu 6 C-Atomen, eine Acylgruppe mit 1 bis 4 C-Atomen oder $R^1$ und $R^2$ zusammen eine Alkylengruppe mit 2 bis 4 C-Atomen, die gegebenenfalls durch eine Hydroxygruppe, eine Alkoxygruppe mit 1 bis 4 C-Atomen oder eine unsubstituierte oder eine durch Benzyl oder Alkyl mit 1 bis 4 C-Atomen mono- oder disubstituierte Aminogruppe substituiert sein kann, bedeuten, X und Y entweder gleich sind und beide jeweils Wasserstoff bedeuten oder X und Y verschieden sind, wobei einer der Reste X oder Y für Wasserstoff steht und der andere von den beiden Resten eine Hydroxy- oder eine Alkoxygruppe mit 1 bis 4 C-Atomen bedeutet, $R^3$, $R^4$, $R^5$, $R^6$, $R^7$, $R^8$, $R^9$ und $R^{10}$ unabhängig voneinander Wasserstoff, $C_{1-4}$-Alkyl, $C_{1-4}$-Alkoxy, $C_{1-4}$-Acyl, Halogen, eine Nitrogruppe, eine unsubstituierte oder durch Benzyl oder Alkylreste mit 1 bis 4 C-Atomen mono- oder disubstituierte Aminogruppe, eine Benzyloxygruppe, eine Hydroxygruppe, eine Aminoalkoxygruppe mit bis zu 12 C-Atomen, am Stickstoffatom unsubstituiert oder mono- oder disubstituiert durch Benzyl oder Alkylreste mit 1 bis 4 C-Atomen oder bei der zwei Substituenten oder ein Substituent am Stickstoffatom mit einem Substituenten der Alkylkette und zusammen mit dem Stickstoffatom einen heterozyklischen Ring mit 3 bis 6 C-Atomen bilden, der auch Sauerstoff-, Schwefel- und/oder weitere Stickstoffatome enthalten und durch Alkylreste mit 1 bis 4 C-Atomen substituiert sein kann, wobei die Alkylkette durch weitere $C_{1-4}$-Alkylreste, eine Hydroxygruppe oder eine $C_{1-4}$-Alkoxygruppe substituiert sein kann, eine Trifluormethylgruppe oder zwei benachbarte Reste zusammen eine Methylendioxygruppe bedeuten, mit der Maßgabe, daß mindestens einer und bis zu vier der Reste $R^3$ bis $R^{10}$ von Wasserstoff verschieden sind, und deren pharmakologisch unbedenkliche Salze.

2. Indolocarbazol-Derivate der allgemeinen Formel I gemäß Anspruch 1, in denen $R^1$ und/oder $R^2$ Wasserstoff, Methyl-, Ethyl-, n-Propyl-, Isopropyl-, n-Butyl-, Cyanomethyl-, 2-Cyanoethyl-, Benzyl-, Acetyl-, Methoxycarbonylmethyl-, 2-Methoxyethyl-, 2-Aminoethyl-, 3-Aminopropyl-, 1-Amino-2-propyl-, 2-Dimethylaminoethyl, 3-Dimethylamino-1-propyl-, 3-Dimethylamino-2-propyl-, 2-Diethylaminoethyl-, 2-[N-Benzyl-N-methylamino]ethyl-, 3-[N-Benzyl-N-methylamino]propyl-, 3-Dimethylamino-2-hydroxy-1-propyl-, 3-Diethylamino-2-hydroxy-1-propyl-, 3-Diethylamino-2-methoxy-1-propyl-, 2-Piperidinoethyl-, 3-Piperidinopropyl-, 2-Pyrrolidinoethyl-, 3-Pyrrolidinopropyl-, 2-Morpholinoethyl, 3-Morpholinopropyl-, 3-Morpholino-2-hydroxy-1-propyl-, Pyrrolidin-2-ylmethyl- oder N-Methyl-pyrrolidin-2-ylmethylgruppen oder $R^1$ und $R^2$ gemeinsam eine Butylen- oder eine hydroxysubstituierte Propylengruppe, $R^3$ bis $R^{10}$ Wasserstoff, Chlor, Brom oder Methyl-, Ethyl-, Hydroxy-, Methoxy-, 2-Aminoethoxy-, 3-Aminopropoxy-, 1-Amino-2-propoxy-, 2-Dimethylaminoethoxy-, 3-Dimethylamino-1-propoxy, 3-Dimethylamino-2-propoxy-, 2-Diethylaminoethoxy-, 2-[N-Benzyl-N-methylamino]ethoxy-, 3-[N-Benzyl-N-methylamino]propoxy-, 3-Dimethylamino-2-hydroxy-1-propoxy-,

2-Piperidinoethoxy-, 3-Piperidinopropoxy-, 2-Pyrrolidinoethoxy-, 3-Pyrrolidino-propoxy-, 2-Morpholinoethoxy-, 3-Morpholinopropoxy-, Pyrrolidin-2-ylmethoxy- oder N-Methylpyrrolidin-2-ylmethoxygruppen und X und Y entweder beide jeweils Wasserstoff oder ein Rest X oder Y Wasserstoff und der andere Rest eine Hydroxy-, Methoxy-, Ethoxy-, Propoxy-, Isopropoxy- oder n-Butoxygruppe darstellt.

3. Verfahren zur Herstellung der Verbindungen der allgemeinen Formel I gemäß Anspruch 1 oder 2, dadurch gekennzeichnet, daß man ein Dibrommaleinimid der allgemeinen Formel IV,

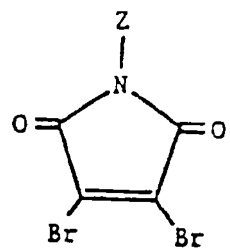

in welchem Z eine geeignete abspaltbare Schutzgruppe ist, mit einem substituierten Indolgrignardreagenz der allgemeinen Formel A,

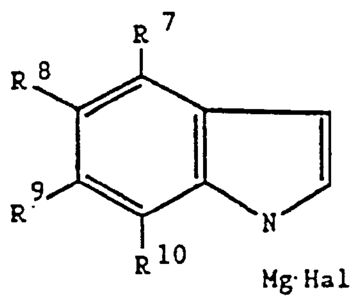

(A)

in welchem $R^7$, $R^8$, $R^9$ und $R^{10}$ die oben genannte Bedeutung haben, umsetzt, und das erhaltene Produkt der allgemeinen Formel V,

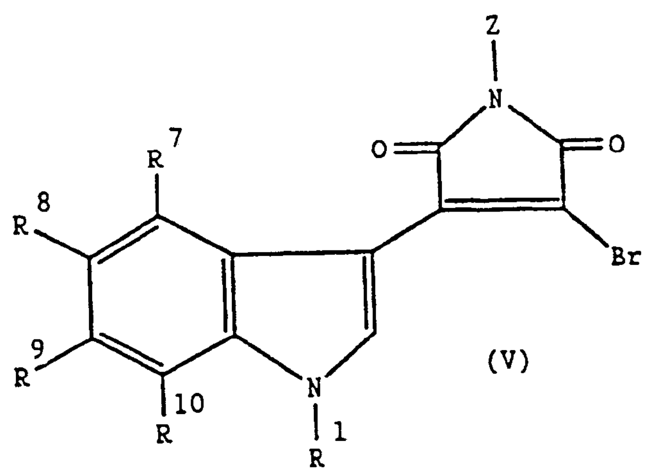

(V)

bei dem $R^1$ Wasserstoff bedeutet, gegebenenfalls am Indolstickstoff mit einem Alkylierungsmittel $R^1$-$X^1$, wobei $R^1$ mit Ausnahme von Wasserstoff die oben genannten Bedeutungen hat und $X^1$ für eine leicht austretende Gruppe wie Chlor oder Brom steht, alkyliert, wobei ein Produkt der allgemeinen Formel V erhalten wird, bei dem $R^1$ verschieden von Wasserstoff ist und anschließend Produkt V mit einem substituierten Indolgrignardreagenz der allgemeinen Formel B,

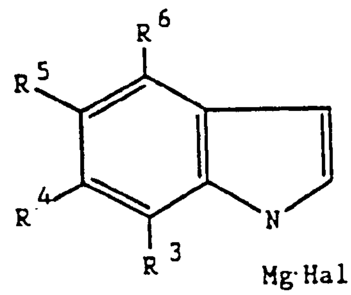

(B)

in welcher $R^3$, $R^4$, $R^5$ und $R^6$ die oben genannte Bedeutung haben, umsetzt und gegebenenfalls anschließend mit einem Alkylierungsmittel der allgemeinen Formel $R^2$-$X^1$, in der $R^2$ mit Ausnahme von Wasserstoff eine der oben genannten Bedeutungen hat und $X^1$ die oben genannte Bedeutung hat, alkyliert zu einem Produkt der allgemeinen Formel VI

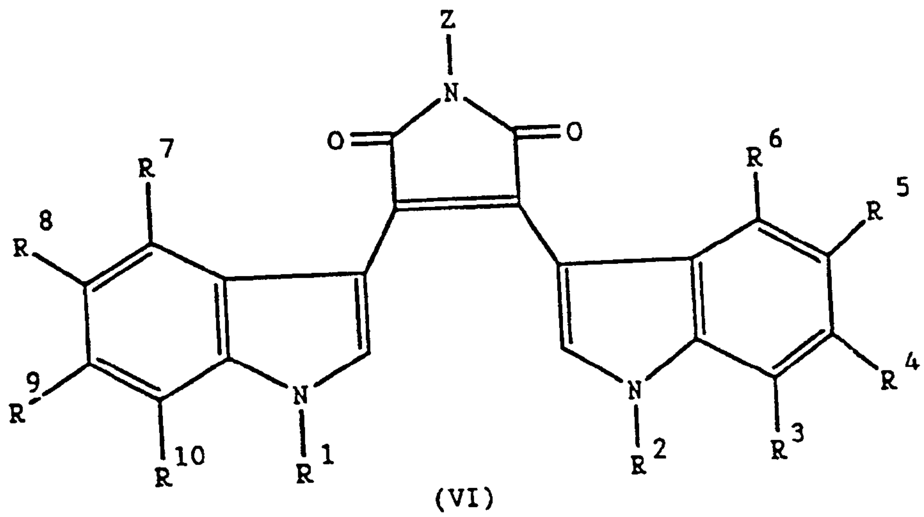

(VI)

und dieses oxidativ cyclisiert zu einer Verbindung der allgemeinen Formel VII,

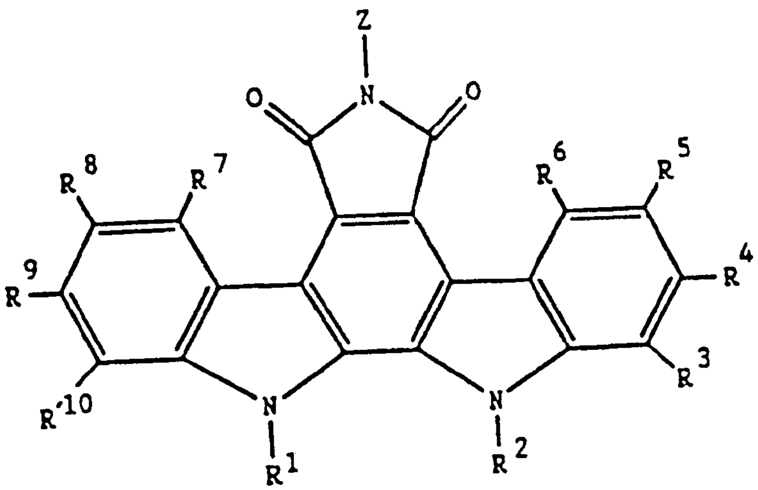

(VII)

worin man die substituierte Imidgruppe in die unsubstituierte Imidgruppe der Verbindung der allgemeinen Formel II

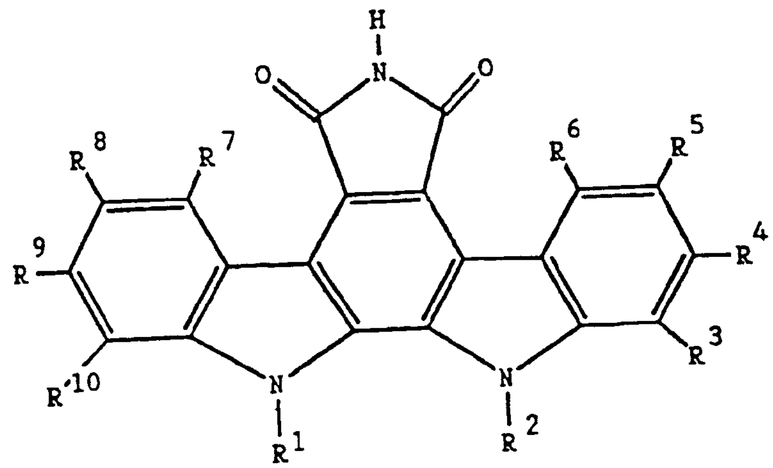

(II)

überführt und man anschließend entweder

A) Imide der allgemeinen Formel II, in welcher $R^1$ bis $R^{10}$ die oben genannte Bedeutung haben, mit Zink oder Zinkamalgam in Gegenwart einer organischen oder anorganischen Säure zu Verbindungen der allgemeinen Formel I, worin X und Y Wasserstoff bedeuten, reduziert, oder

B) Imide der allgemeinen Formel II, in welcher $R^1$ bis $R^{10}$ die oben genannte Bedeutung haben, mit Zink oder Zinkamalgam in Gegenwart einer organischen oder anorganischen Säure, mit Borhydriden oder mit Lithiumaluminiumhydrid zu Verbindungen der allgemeinen Formel I, in welcher X oder Y eine Hydroxygruppe und der jeweils andere Rest Wasserstoff bedeuten, oder, wenn die Reduktion in $C_{1-4}$-Alkoholen durchgeführt wird, auch zu Verbindungen der allgemeinen Formel I, in welcher X oder Y eine $C_{1-4}$-Alkoxygruppe und der jeweils andere Rest Wasserstoff bedeuten, reduziert, oder Verbindungen der allgemeinen Formel I, in welcher X oder Y eine Hydroxygruppe und der jeweils andere Rest Wasserstoff bedeuten, durch säurekatalysierte Umsetzung mit $C_{1-4}$-Alkoholen zu Verbindungen der allgemeinen Formel I, in welcher X oder Y eine $C_{1-4}$-Alkoxygruppe und der jeweils andere Rest Wasserstoff bedeuten, modifiziert, oder

C) Verbindungen der allgemeinen Formel 1, in welcher X, Y, $R^1$ und $R^2$ für Wasserstoff stehen, mit einem oder zwei Äquivalenten einer Verbindung der allgemeinen Formel III,

$$R^{13}\text{-}X^2 \quad \text{(III)}$$

in der $R^{13}$ für eine geradkettige oder verzweigte Alkylgruppe mit 1 bis 18 C-Atomen, eine unsubstituierte oder substituierte Benzylgruppe, eine am Stickstoffatom unsubstituierte oder substituierte Aminoalkylgruppe mit bis zu 12 C-Atomen, wobei die Alkylkette durch weitere $C_{1-4}$-Alkylreste, eine Hydroxygruppe oder eine $C_{1-4}$-Alkoxygruppe substituiert sein kann, einen Alkoxycarbonylalkylrest mit bis zu 6 C-Atomen, einen Halogenalkyl- oder Alkoxyalkylrest mit jeweils bis zu 6 C-Atomen, eine Acylgruppe mit 1 bis 4 C-Atomen und $X^2$ für Halogen steht, in Gegenwart von einem oder zwei Äquivalenten einer Base in an sich bekannter Weise an einem oder beiden Indolstickstoffatomen alkyliert oder acyliert, oder indem man bereits eingeführte Reste $R^{13}$ durch übliche Methoden zu einem der Reste $R^1$ oder $R^2$ modifiziert, oder

D) Verbindungen der allgemeinen Formel I, in welcher X, Y und einer der Reste $R^1$ und $R^2$ Wasserstoff bedeuten, mit einem Äquivalent einer Verbindung der allgemeinen Formel III in Gegenwart einer Base analog Verfahren C alkyliert oder acyliert und gegebenenfalls den eingeführten Rest $R^{13}$ wie oben genannt zu einem der Reste $R^1$ oder $R^2$ modifiziert, oder

E) Verbindungen der allgemeinen Formel I, in der X und Y Wasserstoff bedeuten und $R^1$ und/oder $R^2$ für einen N,N-disubstituierten 3-Amino-2-hydroxy-1-propylrest stehen, durch Alkylierung von Verbindungen der allgemeinen Formel I, in welcher $R^1$ und/oder $R^2$, sowie X und Y für Wasserstoff stehen, mit 1,1-disubstituierten 3-Hydroxyazetidiniumhalogeniden (J. Org. Chem. 1968, 523) herstellt, oder

F) Verbindungen der allgemeinen Formel I, in welcher X und Y Wasserstoff bedeuten und $R^1$ oder $R^2$ für einen 2-Cyanoethylrest oder einen 2-Alkoxycarbonylethylrest mit bis zu 7 C-Atomen steht, durch basenkatalysierte Michael-Addition von Verbindungen der allgemeinen Formel I, in welcher $R^1$ und/oder $R^2$, sowie X und Y für Wasserstoff stehen, an aktivierte Olefine der allgemeinen Formel VIII

$CH_2=CH-R^{14}$ (VIII)

herstellt, in der $R^{14}$ für eine Cyanogruppe oder einen Alkoxycarbonylrest mit bis zu 5 C-Atomen steht, oder

G) Verbindung der allgemeinen Formel I, in welcher X und Y Wasserstoff bedeuten und $R^1$ oder $R^2$ zusammen einen Propylenrest der allgemeinen Formel IX

```
-CH2-CH-CH2
     |          (IX)
     R
```

bilden, in der R für Hydroxy, $C_{1-4}$-Alkoxy oder Amino steht, durch Umsetzung von Verbindungen der allgemeinen Formel I, in welcher $R^1$ und $R^2$, sowie X und Y für Wasserstoff stehen, mit 2 Äquivalenten einer Base und Epichlorhydrin oder Epibromhydrin herstellt, wobei der zunächst gebildete hydroxysubstituierte Propylenrest nach bekannten Methoden in $C_{1-4}$-alkoxy- oder aminosubstituierte Propylenreste übergeführt werden kann.

4. Arzneimittel enthaltend neben üblichen Hilfs- und Zusatzstoffen Verbindungen der allgemeinen Formel I gemäß Anspruch 1.

5. Verwendung von Verbindungen der allgemeinen Formel I gemäß Anspruch 1 zur Herstellung von Pharmazeutika zur Prävention und/oder Behandlung von Herz- und Gefäßkrankheiten wie Thrombosen, Arteriosklerose, Hypertonien, von Entzündungsprozessen, Allergien, Krebs, viralen Erkrankungen und degenerativen Schäden des Zentralnervensystems.

## Claims

1. Indolocarbazole derivatives of the general formula I

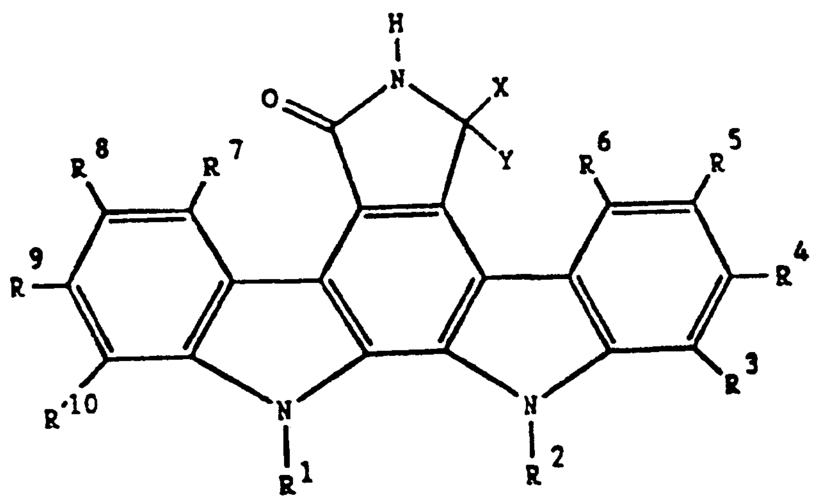

(I)

in which $R^1$ and $R^2$ are the same or different and signify hydrogen, a straight-chain or branched alkyl group with 1 to 18 C-atoms, a cyanoalkyl group with 2 to 4 C-atoms, a benzyl group unsubstituted or substituted with up to three $C_{1-4}$-alkyl groups, $C_{1-4}$-alkoxy groups or halogen atoms, an aminoalkyl group with up to 12 C-atoms, unsubstituted on the nitrogen atom or mono- or disubstituted by benzyl or alkyl radicals with 1 to 4 C-atoms or in which two substituents on the nitrogen atom together with the nitrogen atom or one substituent on the nitrogen atom and one substituent of the alkyl chain and together with the nitrogen atom form a heterocyclic ring with 3 to 6 C-atoms which can also contain oxygen, sulphur and/or further nitrogen atoms and can be substituted by alkyl radicals with 1 to 4 C-atoms, in which case the alkyl chain can be substituted by further $C_{1-4}$-alkyl radicals, a hydroxy group or a $C_{1-4}$-alkoxy group, an alkoxycarbonylalkyl radical with up to 7 C-atoms, a radical $-CH_2-CO-NR^{11}R^{12}$, in which $R^{11}$ and $R^{12}$ are the same or different and stand for hydrogen, an alkyl group with 1 to 6 C-atoms or a benzyl group, a haloalkyl, hydroxyalkyl or alkoxyalkyl radical with, in each case, up to 6 C-atoms, an acyl group with 1 to 4 C-atoms or $R^1$ and $R^2$ together signify an alkylene group with 2 to 4 C-atoms

which can, if applicable, be substituted by a hydroxy group, an alkoxy group with 1 to 4 C-atoms or an amino group unsubstituted or mono- or disubstituted by benzyl or alkyl with 1 to 4 C-atoms, X and Y are either the same, and both in each case signify hydrogen, or X and Y are different, in which case one of the radicals X or Y stands for hydrogen and the other of the two radicals signifies a hydroxy or an alkoxy group with 1 to 4 C-atoms, $R^3$, $R^4$, $R^5$, $R^6$, $R^7$, $R^8$, $R^9$ and $R^{10}$ independently of one another signify hydrogen, $C_{1-4}$-alkyl, $C_{1-4}$-alkoxy, $C_{1-4}$-acyl, halogen, a nitro group, an amino group unsubstituted or mono- or disubstituted by benzyl or alkyl radicals with 1 to 4 C-atoms, a benzyloxy group, a hydroxy group, an aminoalkoxy group with up to 12 C-atoms, unsubstituted on the nitrogen atom or mono- or disubstituted by benzyl or alkyl radicals with 1 to 4 C-atoms or in which two substituents or one substituent on the nitrogen atom with a substituent of the alkyl chain and together with the nitrogen atom form a heterocyclic ring with 3 to 6 C-atoms which can also contain oxygen, sulphur and/or further nitrogen atoms and can be substituted by alkyl radicals with 1 to 4 C-atoms, in which case the alkyl chain can be substituted by further $C_{1-4}$-alkyl radicals, a hydroxy group or a $C_{1-4}$-alkoxy group, a trifluoromethyl group or two neighbouring radicals together signify a methylenedioxy group, provided that at least one and up to four of the radicals $R^3$ to $R^{10}$ are different from hydrogen, and their pharmacologically harmless salts.

2. Indolocarbazole derivatives of the general formula I according to claim 1, in which $R^1$ and/or $R^2$ represent hydrogen, methyl, ethyl, n-propyl, isopropyl, n-butyl, cyanomethyl, 2-cyanoethyl, benzyl, acetyl, methoxycarbonylmethyl, 2-methoxyethyl, 2-aminoethyl, 3-aminopropyl, 1-amino-2-propyl, 2-dimethylaminoethyl, 3-dimethylamino-1-propyl, 3-dimethylamino-2-propyl, 2-diethylaminoethyl, 2-[N-benzyl-N-methylamino]ethyl, 3-[N-benzyl-N-methylamino]propyl, 3-dimethylamino-2-hydroxy-1-propyl, 3-diethylamino-2-hydroxy-1-propyl, 3-diethylamino-2-methoxy-1-propyl, 2-piperidinoethyl, 3-piperidinopropyl, 2-pyrrolidinoethyl, 3-pyrrolidinopropyl, 2-morpholinoethyl, 3-morpholinopropyl, 3-morpholino-2-hydroxy-1-propyl, pyrrolidine-2-ylmethyl or N-methyl-pyrrolidine-2-ylmethyl groups or $R^1$ and $R^2$ together a butylene or a hydroxy-substituted propylene group, $R^3$ to $R^{10}$ hydrogen, chlorine, bromine or methyl, ethyl, hydroxy, methoxy, 2-aminoethoxy, 3-aminopropoxy, 1-amino-2-propoxy, 2-dimetheylaminoethoxy, 3-dimethylamino-1-propoxy, 3-dimethylamino-2-propoxy, 2-diethylaminoethoxy, 2-[N-benzyl-N-methylamino]ethoxy, 3-[N-benzyl-N-methyl-amino]propoxy, 3-dimethylamino-2-hydroxy-1-propoxy, 2-piperidinoethoxy, 3-piperidinopropoxy, 2-pyrrolidino-ethoxy, 3-pyrrolidinopropoxy, 2-morpholinoethoxy, 3-morpholinopropoxy, pyrrolidine-2-ylmethoxy or N-methyl-pyrrolidine-2-ylmethoxy groups and X and Y are either both in each case hydrogen or one radical X or Y represents hydrogen and the other radical a hydroxy, methoxy, ethoxy, propoxy, isopropoxy or n-butoxy group.

3. Process for the preparation of compounds of the general formula I according to claim 1 or 2, characterised in that a dibromomaleinimide of the general formula IV

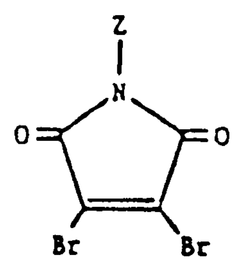

is reacted, in which formula Z is a suitable protective group which can be cleaved, with a substituted indole Grignard reagent of the general formula A

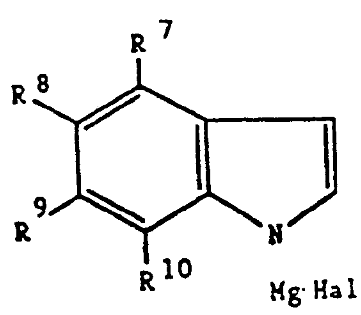

(A)

in which $R^7$, $R^8$, $R^9$ and $R^{10}$ have the above-mentioned signification, and the product obtained of the general

formula V

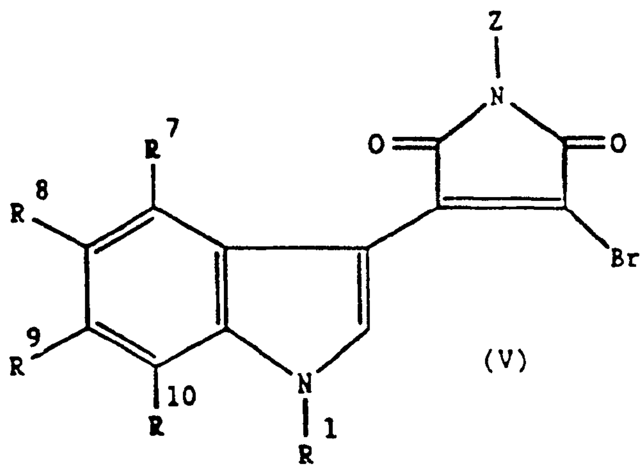

(V)

in which $R^1$ signifies hydrogen, is, if applicable, alkylated on the indole nitrogen with an alkylation agent $R^1$-$X^1$, in which case, with the exception of hydrogen, $R^1$ has the above-mentioned significations and $X^1$ stands for a group which is easily removed, such as chlorine or bromine, in which case a product of the general formula V is obtained in which $R^1$ is different from hydrogen and subsequently product V is reacted with a substituted indole Grignard reagent of the general formula B

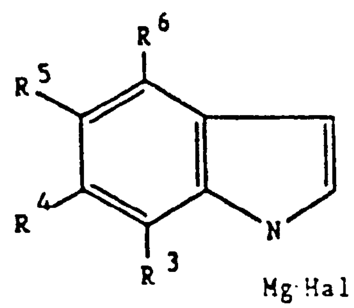

(B)

in which $R^3$, $R^4$, $R^5$ and $R^6$ have the above-mentioned signification and, if applicable, subsequently alkylated with an alkylation agent of the general formula $R^2$-$X^1$, in which, with the exception of hydrogen, $R^2$ has one of the above-mentioned significations and $X^1$ has the above-mentioned signification, to a product of the general formula VI

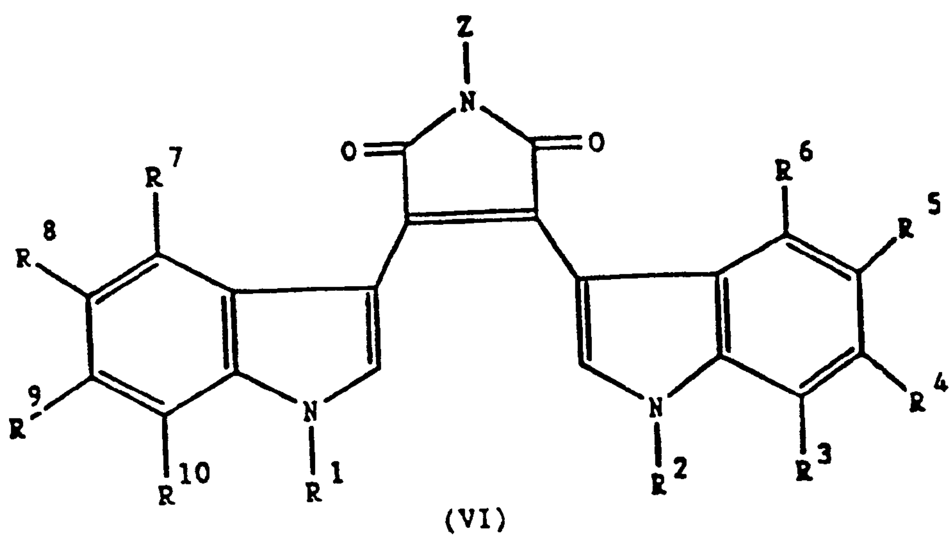

(VI)

and this is cyclized oxidatively to a compound of the general formula VII

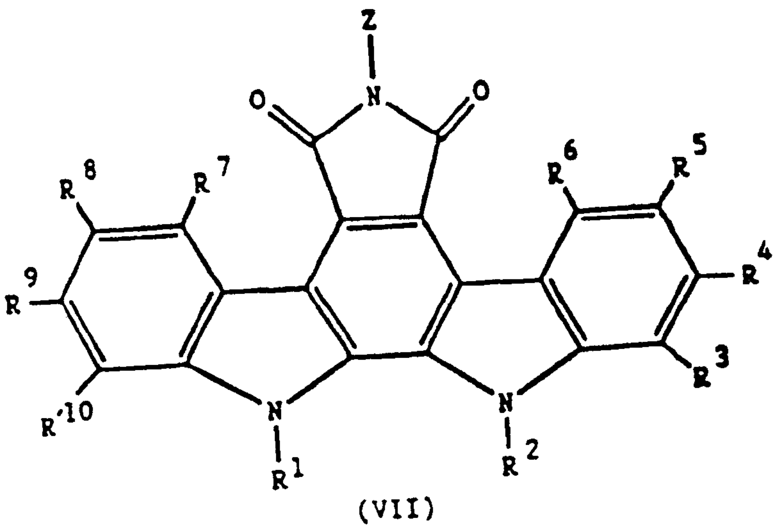

(VII)

wherein the substituted imide group is converted into the unsubstituted imide group of the compound of the general formula II

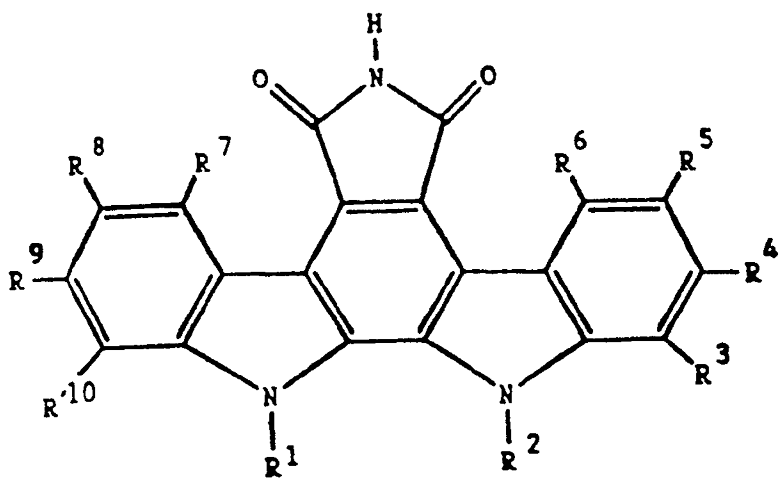

(II)

and subsequently either

A) imides of the general formula II, in which $R^1$ to $R^{10}$ have the above-mentioned signification, are reduced with zinc or zinc amalgam in the presence of an organic or inorganic acid to compounds of the general formula I, wherein X and Y signify hydrogen, or

B) imides of the general formula II, in which $R^1$ to $R^{10}$ have the above-mentioned signification, are reduced with zinc or zinc amalgam in the presence of an organic or inorganic acid, with boron hydrides or with lithium aluminium hydride to compounds of the general formula I, in which X or Y signifies a hydroxy group and the other respective radical hydrogen, or, when the reduction is carried out in $C_{1-4}$-alcohols, also to compounds of the general formula I, in which X or Y signifies a $C_{1-4}$-alkoxy group and the other respective radical hydrogen, or compounds of the general formula I are modified, in which formula X or Y signifies a hydroxy group and the other respective radical hydrogen, by acid-catalysed reaction with $C_{1-4}$-alcohols to compounds of the general formula I, in which X or Y signifies a $C_{1-4}$-alkoxy group and the other respective radical hydrogen, or

C) compounds of the general formula I, in which X, Y, $R^1$ and $R^2$ stand for hydrogen, are alkylated or acylated in a manner known per se, on one or both indole nitrogen atoms, with one or two equivalents of a compound of the general formula III

$$R^{13}\text{-}X^2 \quad (III)$$

in which $R^{13}$ stands for a straight-chain or branched alkyl group with 1 to 18 C-atoms, an unsubstituted or substituted benzyl group, an aminoalkyl group with up to 12 C-atoms unsubstituted or substituted on the nitrogen atom, in which case the alkyl chain can be substituted by further $C_{1-4}$-alkyl radicals, a hydroxy group or a $C_{1-4}$-alkoxy group, an alkoxycarbonyl alkyl radical with up to 6 C-atoms, a haloalkyl or alkoxyalkyl radical with, in each case, up to 6 C-atoms, an acyl group with 1 to 4 C-atoms and $X^2$ for halogen, in the presence of one or two equivalents of a base, or in that already introduced radicals $R^{13}$ are modified by usual methods to one of the radicals $R^1$ or $R^2$, or

D) compounds of the general formula I, in which X, Y and one of the radicals $R^1$ and $R^2$ signify hydrogen, are alkylated or acylated with an equivalent of a compound of the general formula III in the presence of a base analogously to process C and, if applicable, the introduced radical $R^{13}$, as mentioned above, is modified to one of the radicals $R^1$ or $R^2$, or

E) compounds of the general formula I, in which X and Y signify hydrogen and $R^1$ and/or $R^2$ stand for an N,N-disubstituted 3-amino-2-hydroxy-1-propyl radical, are prepared by alkylation of compounds of the general formula I, in which $R^1$ and/or $R^2$, as well as X and Y stand for hydrogen, with 1,1-disubstituted 3-hydroxyazetidinium halides (J. Org. Chem. 1968, 523), or

F) compounds of the general formula I, in which X and Y signify hydrogen and $R^1$ or $R^2$ stands for a 2-cyanoethyl radical or a 2-alkoxycarbonylethyl radical with up to 7 C-atoms, are prepared by base-catalysed Michael-addition of compounds of the general formula I, in which $R^1$ and/or $R^2$, as well as X and Y, stand for hydrogen, on activated olefins of the general formula VIII

$$CH_2{=}CH\text{-}R^{14} \quad (VIII)$$

in which $R^{14}$ stands for a cyano group or an alkoxycarbonyl radical with up to 5 C-atoms, or

G) compound of the general formula I is prepared, in which formula X and Y signify hydrogen and $R^1$ or $R^2$ together form a propylene radical of the general formula IX

$$-CH2-\underset{\displaystyle R}{\underset{|}{CH}}-CH2 \qquad (IX)$$

in which R stands for hydroxy, $C_{1-4}$-alkoxy or amino, by reaction of compounds of the general formula I, in which $R^1$ and $R^2$, as well as X and Y, stand for hydrogen, with 2 equivalents of a base and epichlorohydrin or epibromohydrin, in which case the hydroxy-substituted propylene radical formed first may be converted according to known methods into $C_{1-4}$-alkoxy- or amino-substituted propylene radicals.

4. Medicaments containing, besides usual auxiliary and additive materials, compounds of the general formula I according to claim 1.

5. Use of compounds of the general formula I according to claim 1 for the preparation of pharmaceuticals for the prevention and/or treatment of heart and vascular diseases, such as thromboses, arterioscleroses, hypertonia, of inflammatory processes, allergies, cancers, viral diseases and degenerative damage of the central nervous system.

## Revendications

1. Nouveaux dérivés d'indolocarbazole répondant à la formule générale I :

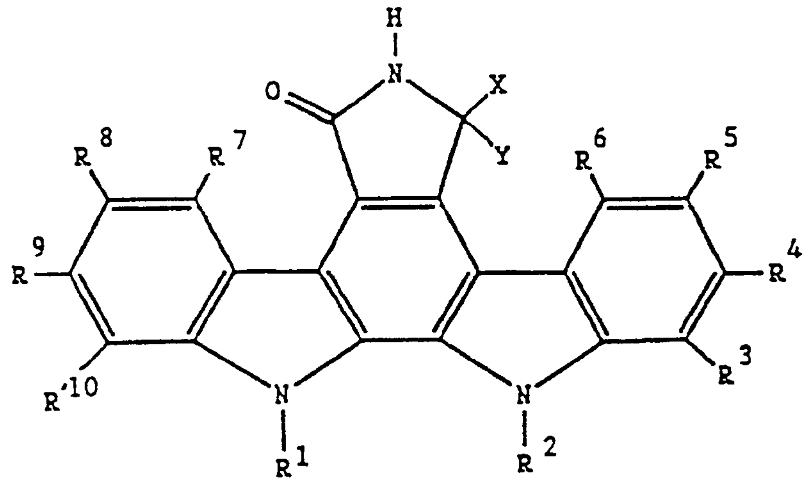

(I)

dans laquelle $R^1$ et $R^2$ sont identiques ou différents et signifient un atome d'hydrogène, un groupe alkyle à chaîne droite ou ramifiée renfermant de 1 à 18 atomes de carbone, un groupe cyanoalkyle renfermant de 2 à 4 atomes de carbone, un groupe benzyle non substitué ou substitué par jusqu'à 3 groupes alkyle en $C_{1-4}$, 3 groupes alcoxy en $C_{1-4}$ ou 3 atomes d'halogène, un groupe aminoalkyle renfermant jusqu'à 12 atomes de carbone, non substitué sur l'atome d'azote ou mono- ou di-substitué par des restes benzyle ou par des restes alkyle renfermant de 1 à 4 atomes de carbone ou dans lequel deux substituants sur l'atome d'azote, en même temps que l'atome d'azote, ou un substituant sur l'atome d'azote et un substituant de la chaîne alkyle, et en même temps que l'atome d'azote forment un cycle hétérocyclique renfermant de 3 à 6 atomes de carbone qui peut également contenir d'autres atomes d'oxygène, de soufre et/ou d'autres atomes d'azote et peut être substitué par des radicaux alkyle renfermant de 1 à 4 atomes de carbone, la chaîne alkyle pouvant être substituée par d'autres restes alkyle en $C_{1-4}$, un groupe hydroxy ou un groupe alcoxy en $C_{1-4}$, un reste alcoxycarbonylalkyle renfermant jusqu'à 7 atomes de carbone, un reste $-CH_2-CO-NR^{11}R^{12}$ dans lequel $R^{11}$ et $R^{12}$ sont identiques ou différents et représentent un atome d'hydrogène, un groupe alkyle renfermant de 1 à 6 atomes de carbone ou un groupe benzyle, un reste halogénoalkyle, un reste hydroxyalkyle ou un reste alcoxyalkyle renfermant chaque fois jusqu'à 6 atomes de carbone, un groupe acyle renfermant de 1 à 4 atomes de carbone ou bien $R^1$ et $R^2$ signifient ensemble un groupe alkylène renfermant de 2 à 4 atomes de carbone pouvant le cas échéant être substitué par un groupe hydroxy, un groupe alcoxy renfermant de 1 à 4 atomes de carbone ou un groupe amino non substitué ou mono- ou disubstitué par des radicaux benzyle ou des radicaux alkyle renfermant de 1 à 4 atomes de carbone, X et Y sont soit identiques et signifient tous les deux un atome d'hydrogène, ou X et Y sont différents, l'un des restes de X et Y signifiant un atome d'hydrogène et l'autre des deux restes signifiant un groupe hydroxy ou un groupe alcoxy renfermant de 1 à 4 atomes de carbone, $R^3$, $R^4$, $R^5$, $R^6$, $R^7$, $R^8$, $R^9$ et $R^{10}$ signifient indépendamment les uns des autres un atome d'hydrogène, un radical alkyle en $C_{1-4}$, alcoxy en $C_{1-4}$, acyle en $C_{1-4}$, un atome d'halogène, un groupe nitro, un groupe amino non substitué ou disubstitué par des restes benzyle ou des restes alkyle renfermant de 1 à 4 atomes de carbone, un groupe benzyloxy, un groupe hydroxy, un groupe aminoalcoxy renfermant jusqu'à 12 atomes de carbone, non substitué sur l'atome d'azote ou mono- ou disubstitué par des restes benzyle ou des restes alkyle renfermant de 1 à 4 atomes de carbone ou dans lequel deux substituants ou un substituant sur l'atome d'azote en même temps qu'un substituant de la chaîne alkyle et en même temps que l'atome d'azote forment un cycle hétérocyclique renfermant de 3 à 6 atomes de carbone pouvant également contenir des atomes d'oxygène, de soufre et/ou d'autres atomes d'azote et pouvant être substitué par des radicaux alkyle renfermant de 1 à 4 atomes de carbone, la chaîne alkyle pouvant être substituée par d'autres radicaux alkyle en $C_{1-4}$, un groupe hydroxy ou un groupe alcoxy en $C_{1-4}$, un groupe trifluorométhyle ou deux restes voisins signifiant en même temps un groupe méthylènedioxy, sous réserve qu'au moins l'un et jusqu'à quatre des restes $R^3$ à $R^{10}$ sont différents d'un atome d'hydrogène, et leurs sels pharmacologiquement compatibles.

2. Dérivés d'indolocarbazole de formule générale I selon la revendication 1, dans lesquels $R^1$ et/ou $R^2$ représentent un atome d'hydrogène, un radical méthyle, éthyle, n-propyle, isopropyle, n-butyle, cyanométhyle, 2-cyanoéthyle, benzyle, acétyle, méthoxycarbonylméthyle, 2-méthoxyéthyle, 2-aminoéthyle, 3-aminopropyle, 1-amino-2-propyle, 2-diméthylaminoéthyle, 3-diméthylamino-1-propyle, 3-diméthylamino-2-propyle, 2-diéthylaminoéthyle, 2-[N-benzyl-N-méthylamino]-éthyle, 3-[N-benzyl-N-méthylamino]propyle, 3-diméthylamino-2-hydroxy-1-propyle, 3-diéthylamino-2-hydroxy-1-propyle, 3-diéthylamino-2-méthoxy-1-propyle, 3-pipéridinopropyle, 2-pipéridinoéthyle, 2-pyrrolidinoéthyle, 3-pyrrolidinopropyle, 2-morpholino-éthyle, 3-morpholinopropyle, 3-morpholino-2-hydroxy-1-propyle, pyrrolidin-2-ylméthyle ou N-méthyl-pyrrolidin-2-ylméthyle ou $R^1$ et $R^2$ forment ensemble un groupe butylène ou un groupe propylène hydroxy-substitué, $R^3$ à $R^{10}$ signifient un atome d'hydrogène, de chlore, de brome ou un groupe hydroxy, méthyle, éthyle, méthoxy, 2-aminoéthoxy, 3-aminopropoxy, 1-amino-2-propoxy, 2-diméthylaminoéthoxy, 3-diméthylamino-1-propoxy, 3-diméthylamino-2-propoxy, 2-diéthylaminoéthoxy, 2-[N-benzyl-N-méthylamino]éthoxy, 3-[N-benzyl-N-méthyl-amino]propoxy, 3-diméthylamino-2-hydroxy-1-propoxy, 2-pipéridinoéthoxy, 3-pipéridinopropoxy, 2-pyrrolidino-éthoxy, 3-pyrrolidino-propoxy, 2-morpholinoéthoxy, 3-morpholinopropoxy, pyrrolidin-2-ylméthoxy ou N-méthyl-pyrrolidin-2-ylméthoxy et X et Y signifient soit tous les deux chaque fois un atome d'hydrogène ou un reste X ou Y signifie un atome d'hydrogène et l'autre reste signifie un groupe hydroxy, méthoxy, éthoxy, propoxy, isopropoxy ou n-butoxy.

3. Procédé de préparation des dérivés de formule générale I selon la revendication 1 ou 2, caractérisé en ce que l'on fait réagir un dibromomaléinimide de formule générale IV

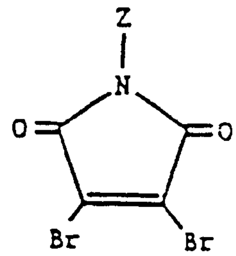

dans laquelle Z représente un groupe protecteur partant convenable avec un réactif de Grignard à base d'indole substitué répondant à la formule générale A

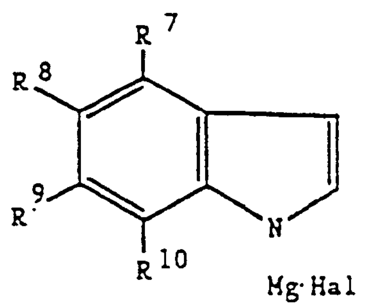

(A)

dans laquelle $R^7$, $R^8$, $R^9$ et $R^{10}$ présentent la signification indiquée ci-dessus et le produit obtenu de formule générale V

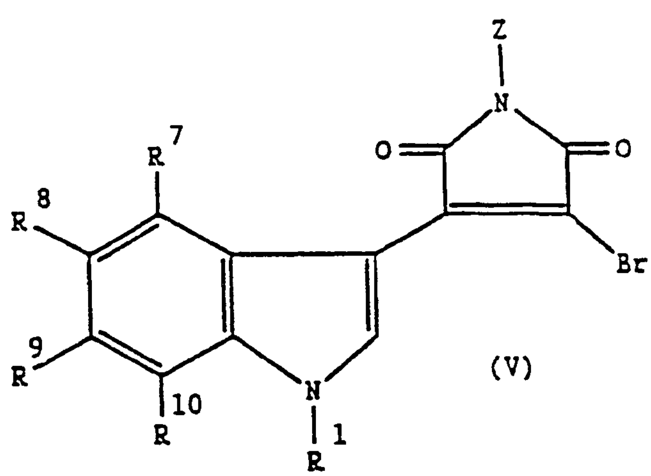

(V)

dans laquelle $R^1$ représente un atome d'hydrogène et soumis le cas échéant à une alkylation sur l'atome d'indole à l'aide d'un agent d'alkylation $R^1$-$X^1$, $R^1$ présentant les significations indiquées ci-dessus à l'exception

de l'hydrogène et $X^1$ représentant un groupe partant léger tel que chlore ou brome, et l'on obtient un produit répondant à la formule générale V dans laquelle $R^1$ est différent de l'hydrogène et on fait ensuite réagir le produit V avec un réactif de Grignard à base d'indole substitué répondant à la formule générale B

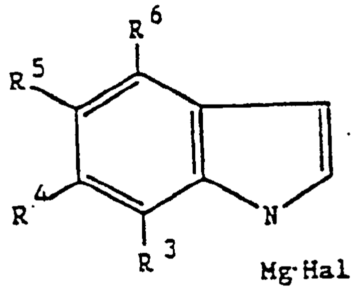
(B)

dans laquelle $R^3$, $R^4$, $R^5$ et $R^6$ présentent la signification indiquée ci-dessus et le cas échéant on soumet à une alkylation à l'aide d'un agent d'alkylation répondant à la formule générale $R^2$-$X^1$ dans laquelle $R^2$ présente l'une des significations indiquées ci-dessus à l'exception de l'hydrogène et $X^1$ présente la signification indiquée ci-dessus pour obtenir un produit de formule générale VI

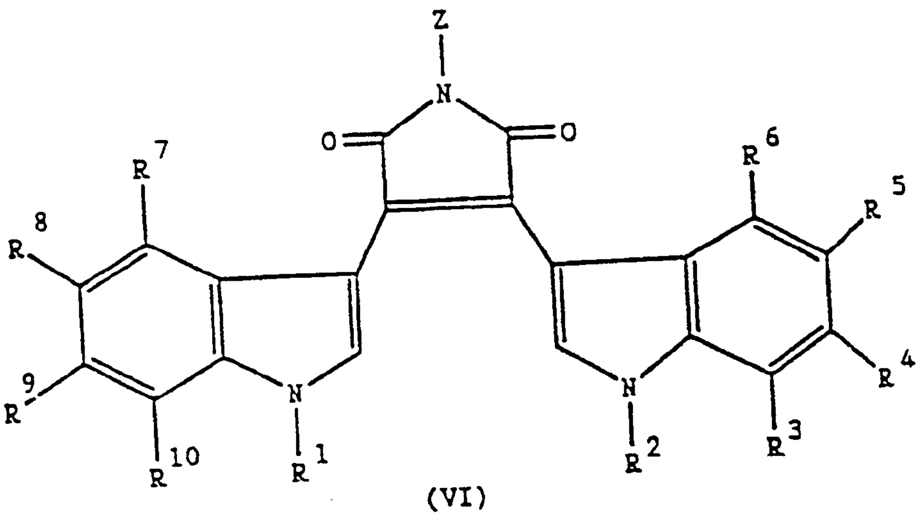
(VI)

et on soumet celui-ci à une cyclisation par oxydation pour obtenir un dérivé de formule générale VII

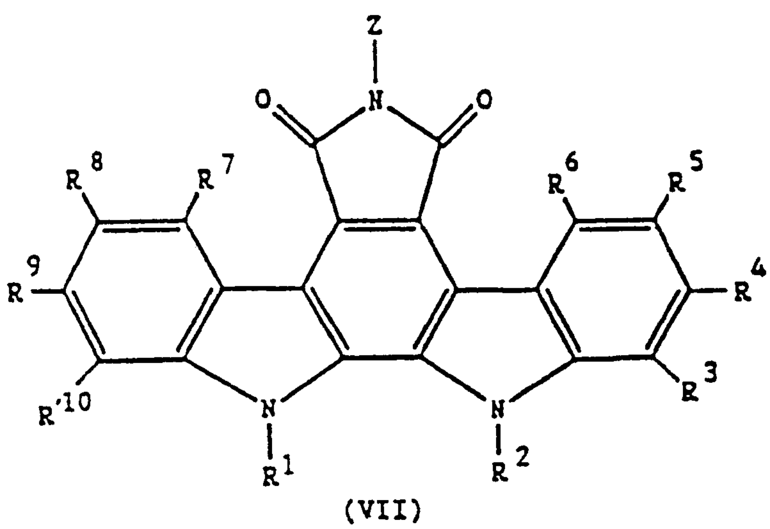
(VII)

et on transforme le groupe imide substitué en groupe imide non substitué du dérivé de formule générale II

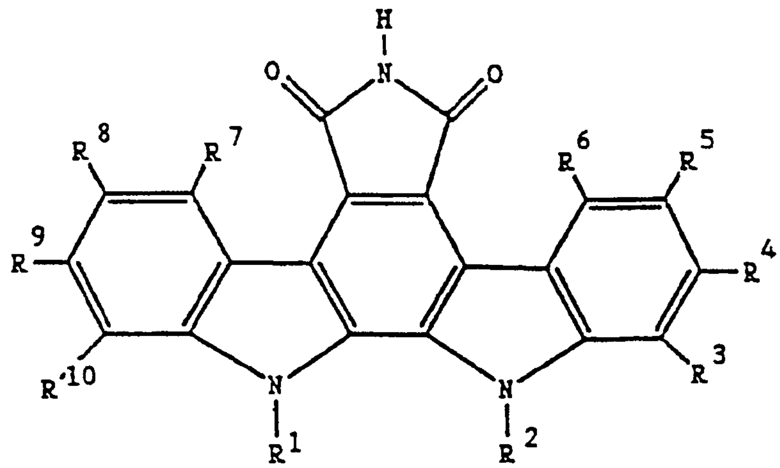

(II)

et ensuite, soit

A) on réduit les imides de formule générale II dans laquelle $R^1$ à $R^{10}$ présentent la signification indiquée ci-dessus, à l'aide de zinc ou d'amalgame de zinc en présence d'un acide organique ou minéral en dérivés de formule générale I dans lesquels X et Y représentent un atome d'hydrogène, ou bien

B) on réduit des imides de formule générale II dans laquelle $R^1$ à $R^{10}$ présentent la signification indiquée ci-dessus à l'aide de zinc ou d'amalgame de zinc en présence d'un acide organique ou minéral à l'aide d'hydrures de bore ou à l'aide d'hydrures de lithiumaluminium en dérivés de formule générale I dans laquelle X ou Y représente un groupe hydroxy et l'autre groupe représente chaque fois un atome d'hydrogène ou bien, lorsque la réduction est mise en oeuvre dans des alcools en $C_{1-4}$, également en dérivés de formule générale I dans laquelle X ou Y représente un groupe alcoxy en $C_{1-4}$ et l'autre reste signifie un atome d'hydrogène, ou bien on modifie des dérivés de formule générale I dans laquelle X ou Y représente un groupe hydroxy et l'autre reste représente chaque fois un atome d'hydrogène, par réaction catalysée par un acide à l'aide d'alcools en $C_{1-4}$, en dérivés de formule générale I dans laquelle X ou Y signifie un groupe alcoxy en $C_{1-4}$ et l'autre reste signifie chaque fois un atome d'hydrogène ou bien

C) on procède à une alkylation ou à une acylation de dérivés de formule générale I dans laquelle X, Y, $R^1$ et $R^2$ représentent un atome d'hydrogène, à l'aide d'un ou de deux équivalents d'un dérivé de formule générale III

$$R^{13}\text{-}X^2 \quad (III)$$

dans laquelle $R^{13}$ signifie un groupe alkyle à chaîne droite ou ramifiée renfermant de 1 à 18 atomes de carbone, un groupe benzyle non substitué ou substitué, un groupe aminoalkyle non substitué sur l'atome d'azote ou substitué par jusqu'à 12 atomes de carbone, la chaîne alkyle pouvant être substituée par d'autres restes alkyle en $C_{1-4}$, un groupe hydroxy ou un groupe alcoxy en $C_{1-4}$, un reste alcoxycarbonylalkyle renfermant jusqu'à 6 atomes de carbone, un reste halogénoalkyle ou un reste alcoxyalkyle renfermant chaque fois jusqu'à 6 atomes de carbone, un groupe acyle renfermant de 1 à 4 atomes de carbone et $X^2$ représente de préférence un atome d'halogène, en présence d'un ou de deux équivalents d'une base d'une façon connue en soi sur un ou deux des atomes d'azote de l'indole, ou bien dans lequel on modifie les restes $R^{13}$ déjà introduits par des méthodes habituelles de la chimie organique, en le reste $R^1$ ou $R^2$, ou bien

D) on procède à une alkylation ou à une acylation des dérivés de formule générale I dans laquelle X, Y et l'un des restes $R^1$ et $R^2$ signifient un atome d'hydrogène, à l'aide d'un équivalent d'un dérivé de formule générale III en présence d'une base de façon analogue à celle employée dans le procédé C et le cas échéant on modifie le reste $R^{13}$ introduit, pour obtenir l'un des restes $R^1$ ou $R^2$, ou bien

E) on prépare des dérivés de formule générale I dans laquelle X et Y signifient un atome d'hydrogène et $R^1$ et/ou $R^2$ représentent un reste N,N-disubstitué 3-amino-2-hydroxy-1-propyle par alkylation de dérivés de formule générale I dans laquelle $R^1$ et/ou $R^2$ ainsi que X et Y représentent un atome d'hydrogène à l'aide d'halogénure de 1,1-disubstitué 3-hydroxyazétidinium (J. Org. Chem. 1968, 523), ou bien

F) on prépare des dérivés de formule générale I dans laquelle X et Y signifient un atome d'hydrogène et

$R^1$ ou $R^2$ représente un reste 2-cyanoéthyle ou un reste 2-alcoxycarbonyléthyle renfermant jusqu'à 7 atomes de carbone par une addition de Michael catalysée par des bases de dérivés de formule générale I dans laquelle $R^1$ et/ou $R^2$ ainsi que X et Y représentent un atome d'hydrogène sur des oléfines activées répondant à la formule générale VIII

$$CH_2=CH-R^{14} \quad (VIII)$$

alcoxycarbonyle renfermant jusqu'à 5 atomes de carbone, ou bien

G) on prépare un dérivé de formule générale I dans laquelle X et Y signifient un atome d'hydrogène et $R^1$ ou $R^2$ forment ensemble un reste propylène de formule générale IX

$$-CH_2-CH-CH_2 \qquad (IX)$$

|

R

dans laquelle R représente un groupe hydroxy, alcoxy en $C_{1-4}$ ou amino, par réaction des dérivés de formule générale I dans laquelle $R^1$ et $R^2$ ainsi que X et Y représentent un atome d'hydrogène, à l'aide de deux équivalents d'une base et d'épichlorhydrine ou d'épibromhydrine, le reste propylène hydroxy-substitué tout d'abord formé pouvant être transformé par des méthodes connues en restes propylène substitués par un groupe alcoxy en $C_{1-4}$ ou un groupe amino.

4. Médicament contenant, outre les substances auxiliaires et les additifs usuels, des dérivés de formule générale I selon la revendication 1.

5. Utilisation de dérivés de formule générale I selon la revendication 1 pour la préparation de produits pharmaceutiques destinés à la prévention et/ou au traitement de maladies cardiaques et vasculaires telles que thrombose, artériosclérose, hypertension, le traitement de processus inflammatoires, d'allergies, de cancers, de maladies virales et de désordres dégénératifs du système nerveux central.